(19) **Europäisches Patentamt**
**European Patent Office**
**Office européen des brevets**

(11) **EP 4 667 559 A1**

(12) **EUROPEAN PATENT APPLICATION**
published in accordance with Art. 153(4) EPC

(43) Date of publication:
**24.12.2025  Bulletin 2025/52**

(21) Application number: **23953304.5**

(22) Date of filing: **25.09.2023**

(51) International Patent Classification (IPC):
**C12N 1/16** (2006.01)    **C12P 7/64** (2022.01)
**C12R 1/645** (2006.01)

(52) Cooperative Patent Classification (CPC):
**C12N 1/16; C12P 7/64;** C12R 2001/645

(86) International application number:
**PCT/CN2023/121022**

(87) International publication number:
**WO 2025/065137 (03.04.2025 Gazette 2025/14)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Wanhua Chemical Group Co., Ltd.**
**Yantai, Shandong 264006 (CN)**

(72) Inventors:
• **WANG, Bei**
  **Shandong 264006 (CN)**
• **MA, Guang**
  **Shandong 264006 (CN)**
• **PAN, Zhaoyang**
  **Shandong 264006 (CN)**
• **ZHANG, Yaping**
  **Shandong 264006 (CN)**
• **MA, Lingyan**
  **Shandong 264006 (CN)**
• **ZHANG, Yongzhen**
  **Shandong 264006 (CN)**

(74) Representative: **Potter Clarkson**
**Chapel Quarter**
**Mount Street**
**Nottingham NG1 6HQ (GB)**

(54) **METHOD FOR PRODUCING SINGLE-CELL PROTEIN BY MEANS OF HIGH-DENSITY FERMENTATION**

(57)    Disclosed herein is a method for producing a single-cell protein by means of high-density fermentation. The method comprises: inoculating a Yarrowia lipolytica strain into a seed culture medium for seed culture to obtain a seed liquid; and inoculating the seed liquid into a fermentation culture medium for high-density fermentation, and supplementing same with acetic acid and a nitrogen source during the process of high-density fermentation to obtain the single-cell protein, wherein the carbon source of the seed culture medium comprises a first acetate, and the carbon source of the fermentation culture medium comprises a second acetate and/or a ketone compound. In the present application, the compounding of specific materials and processes effectively increases the growth rate of bacteria and the synthesis rate of single-cell proteins, so that the method can realize the high-density fermentation of bacteria and efficient synthesis of single-cell proteins in a short time, a fermentation product with a higher content of crude proteins can be obtained while the culture time of high-density fermentation is significantly shortened, and the production efficiency and yield of single-cell proteins are greatly improved.

FIG.1

EP 4 667 559 A1

## Description

### TECHNICAL FIELD

The embodiments of the present application relate to the field of biotechnology, such as a method for producing single-cell protein by means of high-density fermentation.

### BACKGROUND

Currently, with the continuous growth of population, people's consumption demand for food, especially meat, is constantly increasing. It is estimated that by 2050, the global demand for meat will exceed 400 million tons. As the world's most populous country, China's demand for food is also continuously increasing. In 2018, China's meat consumption reached 88.296 million tons, surpassing the combined total of the European Union and the United States. Alongside the increase in meat consumption, the demand for feed is also expanding continuously, with feed production exceeding 240 million tons. The main components of feed are primarily starch and protein, with the primary sources of protein being plant-based protein (e.g., soybean meal) and animal-based protein (e.g., fish meal). Soybean meal, which is a by-product obtained after extracting soybean oil from soybeans, exhibits the highest production and the most extensive application among 12 types of animal and plant oil meal feed products, such as cottonseed meal, peanut meal and rapeseed meal, etc. The production of soybean meal is related to the production of soybean, however, China's self-sufficiency rate in soybeans is insufficient, with about 85% relying on imports. The shortage of soybeans has implications for both food security and protein security, necessitating the exploration of alternative resources available for feed. Furthermore, the supply of animal-based protein such as fish meal in feed is also continuously tightening. In 2020, the global consumption of fish meal was 3 million tons, and it is estimated that by 2025, the shortage of fish meal may reach 1 million tons. China's annual production of fish meal is about 1.2 million tons, with a self-sufficiency rate of about 40%-50%. In 2020, China's fish meal imports reached 1.42 million tons, with an import value of 15 billion. In addition to the shortage problem, the large-scale production and overfishing of fish meal have also greatly overdrawn marine fishery resources, resulting in a decrease in fish number and population, which has a significant impact on ecological health and safety. Based on the above considerations, low-protein feed and unconventional protein substitutes have become the focus of attention in animal husbandry, among which single-cell protein (SCP) is the most important and promising protein substitute.

Single-cell protein, also known as microbial protein, refers to microbial thallus artificially cultivated from various industrial and agricultural wastes as well as petroleum wastes. Single-cell protein is not a pure protein, but rather a cytoplasmic mass composed of a mixture of proteins, fats, carbohydrates, nucleic acids, non-protein nitrogen-containing compounds, vitamins and inorganic compounds. The chemical composition is generally dominated by proteins and fats.

The application of single-cell protein in feed began around the 1990s. A series of animal experiments were carried out on poultry and livestock, aquatic products, and pigs using monosodium glutamate bacteria residue and yeast bacteria residue, etc. The results indicated that thallus protein had certain benefits for animal health, thus initiating the application of single-cell protein in the field of feed. Currently, yeast has been widely used in poultry, livestock and aquatic product feed, accounting for about 5% to 10% of all feed production. The proportion of single-cell protein in aquatic feed is even higher, typically accounting for about 10% to 20%. With the development of technology and processes, proteins derived from Clostridium autoethanogenum and methanogen have also been developed. Although animal experimental data indicate the safety and bioavailability of these proteins, due to the novelty of the technology, no feed products containing Clostridium autoethanogenum and methanogen have been launched on the market yet. In addition, photosynthetic bacteria and algae also have certain applications in feed protein, but the production is relatively small and they are not mainstream products.

Single-cell protein is typically prepared using bio-fermentation technology, which includes the following steps: after the preparation and sterilization of the culture medium are completed, the culture medium is placed into a fermentation tank along with the strain, and fermentation conditions are controlled to allow rapid proliferation of the strain; upon completion of fermentation, the thallus are collected through methods such as centrifugation and precipitation, and finally dried to produce the finished single-cell protein product. Preparing single-cell protein through synthetic biology fermentation method allows for the fixation of $CO_2$ through biological transformation, yielding thallus protein for use in feed, which is beneficial for alleviating resource shortages caused by the relatively high demand for meat and environmental pollution issues caused by excessive use of fish meal. Currently, the country is vigorously promoting low-protein feed and unconventional protein resources, and researchers in agriculture and animal husbandry are also vigorously promoting the promotion of single-cell protein. Single-cell protein is an important development direction in the field of feed additives in the future. However, the current fermentation and production cycle of single-cell protein is long, and there are significant shortcomings in both yield and production capacity, making it difficult to meet the needs of large-scale applications. Therefore, developing preparation methods for single-cell protein with higher efficiency and higher yield is a research focus in the art.

## SUMMARY OF THE INVENTION

The following is a summary of the subject described in detail in the present application. This summary is not intended to limit the scope of protection of the claims.

The examples of the present application provide a method for producing single-cell protein by means of high-density fermentation. By designing carbon sources and adopting the compounding of specific materials and processes, the method can realize the high-density fermentation of thallus and efficient synthesis of single-cell proteins in a short time, greatly improving the production efficiency and yield of single-cell protein.

The examples of the present application provide a method for producing single-cell protein by means of high-density fermentation. The method includes: inoculating a Yarrowia lipolytica strain into a seed culture medium for seed culture to obtain a seed liquid; inoculating the seed liquid into a fermentation culture medium for high-density fermentation, and supplementing same with acetic acid and a nitrogen source during the process of high-density fermentation to obtain the single-cell protein; the carbon source of the seed culture medium comprises a first acetate, and the carbon source of the fermentation culture medium comprises a second acetate and/or a ketone compound.

The present application uses Yarrowia lipolytica strain as the fermentation strain, which is a non-genetically engineered strain and is safer than conventional thallus. Yarrowia lipolytica is a microorganism generally recognized as safe (GRAS) by the US Food and Drug Administration (FDA), making it ideal strain for use in the food industry. The method involves designing the carbon source for seed culture and high-density fermentation. The addition of sodium acetate to the seed culture medium enable promoting the formation of a unique acetic acid metabolic pathway in the thallus, laying the foundation for faster growth during subsequent high-density fermentation when acetic acid is added. Meanwhile, sodium acetate is also included in the carbon source of the fermentation culture medium to further induce and promote the acetic acid metabolic pathway in the thallus. During the high-density fermentation process, acetic acid and nitrogen sources are supplemented. On one hand, wherein the acetic acid can neutralize alkaline substances produced during fermentation, allowing the thallus to grow in an optimal neutral pH environment. On the other hand, as a carbon source, acetic acid has a high carbon content, can effectively promoting rapid growth of thallus and the synthesis of single-cell proteins. Moreover, acetic acid does not require sterilization and can be directly added. Furthermore, the fermentation culture medium contains a ketone fermentation promoter, which has catalytic functions on the intermediate products in the acetic acid metabolic pathway, thereby promoting rapid growth of the thallus and shortening the fermentation period.

In summary, in the method provided by the present application, by introducing the first acetate into the carbon source of the seed culture medium, the formation of a unique acetic acid metabolic pathway of thallus is induced and promoted, thereby laying the foundation for the subsequent supplementation of acetic acid as a carbon source and promoting rapid growth of the thallus during high-density fermentation. Meanwhile, the carbon source of the fermentation culture medium includes a second acetate and/or a ketone compound, which promote rapid growth of the thallus. During the high-density fermentation stage, a nitrogen source and acetic acid are supplemented. While regulating the thallus to be in an optimal neutral pH growth environment, appropriate nutrients are supplied to the thallus to support their continuous growth, thereby effectively improving the growth rate of the thallus and the synthesis rate of single-cell protein. Through the compounding of specific materials and processes, the method can realize high-density fermentation of thallus and efficient synthesis of single-cell proteins in a short period of time, a fermentation product with a higher content of crude proteins can be obtained while the culture time of high-density fermentation is significantly shortened, and the production efficiency and yield of single-cell proteins are greatly improved.

The following serves as the preferred technical solution of the present application, but not a limitation on the technical solutions provided by the present application. Through the following preferred technical solutions, the purpose and beneficial effects of the present application can be better achieved and realized.

Preferably, the first acetate includes any one or a combination of at least two of sodium acetate, potassium acetate and ammonium acetate, further preferably sodium acetate.

Preferably, the mass concentration of the first acetate in the seed culture medium is in a range from 5 g/L to 15 g/L, for example, it may be 6 g/L, 7 g/L, 8 g/L, 9 g/L, 10 g/L, 11 g/L, 12 g/L, 13 g/L or 14 g/L, etc.

Preferably, the seed culture includes a primary seed culture and a secondary seed culture carried out sequentially.

Preferably, a primary seed culture medium used for the primary seed culture includes the following components: 5-15 g/L of first acetate, 1%-3% of glucose, 0.6%-2% of yeast powder and 1.5%-3.2% of casein peptone.

Specifically, the mass concentration of the first acetate (preferably sodium acetate) in the primary seed culture medium is in a range from 5 g/L to 15 g/L, for example, it may be 6 g/L, 7 g/L, 8 g/L, 9 g/L, 10 g/L, 11 g/L, 12 g/L, 13 g/L or 14 g/L, etc.

The mass percentage content of glucose (anhydrous glucose) in the primary seed culture medium is in a range from 1% to 3%, for example, it may be 1.2%, 1.5%, 1.8%, 2%, 2.2%, 2.5% or 2.8%, etc.

The mass percentage content of yeast powder in the primary seed culture medium is in a range from 0.6% to 2%, for example, it may be 0.8%, 1%, 1.2%, 1.5% or 1.8%, etc.

The mass percentage content of casein peptone in the primary seed culture medium is in a range from 1.5% to 3.2%, for example, it may be 1.6%, 1.8%, 2%, 2.2%, 2.5%, 2.8% or 3%, etc.

Preferably, the remainder of the primary seed culture medium is water.

Preferably, the temperature for the primary seed culture is in a range from 30°C to 33°C, for example, it may be 30.2°C, 30.5°C, 30.8°C, 31°C, 31.2°C, 31.5°C, 31.8°C, 32°C, 32.2°C, 32.5°C or 32.8°C, etc., further preferably 31.5°C to 32.5°C.

Preferably, the duration of the primary seed culture is in a range from 16 h to 18 h, for example, it may be 16.2 h, 16.5 h, 16.8 h, 17 h, 17.2 h, 17.5 h or 17.8 h, etc.

Preferably, the primary seed culture is carried out under stirring conditions, with the stirring speed ranging from 100 rpm to 300 rpm, for example, it may be 120 rpm, 150 rpm, 180 rpm, 200 rpm, 220 rpm, 250 rpm or 280 rpm, etc. Further preferably, the stirring speed is 170 rpm to 190 rpm.

Preferably, the primary seed culture is processed to an OD value of 4.2 to 6.8, such as an OD value of 4.3, 4.5, 4.8, 5, 5.2, 5.5, 5.8, 6, 6.2, 6.5 or 6.7, etc., obtaining the primary seed.

Preferably, a secondary seed culture medium used for the secondary seed culture includes the following components: 5-20 g/L of first acetate, 1%-3% of glucose, 0.6%-2% of yeast powder, 1.5%-3.2% of casein peptone, 0.8%-2.4% of magnesium sulfate, and 0.2%-0.8% of thiamine hydrochloride.

Specifically, the mass concentration of the first acetate (preferably sodium acetate) in the secondary seed culture medium is in a range from 5 g/L to 20 g/L, for example, it may be 6 g/L, 8 g/L, 10 g/L, 11 g/L, 13 g/L, 15 g/L, 17 g/L or 19 g/L, etc.

The mass percentage content of glucose (anhydrous glucose) in the secondary seed culture medium is in a range from 1% to 3%, for example, it may be 1.2%, 1.5%, 1.8%, 2%, 2.2%, 2.5% or 2.8%, etc.

The mass percentage content of yeast powder in the secondary seed culture medium is in a range from 0.6% to 2%, for example, it may be 0.8%, 1%, 1.2%, 1.5% or 1.8%, etc.

The mass percentage content of casein peptone in the secondary seed culture medium is in a range from 1.5% to 3.2%, for example, it may be 1.6%, 1.8%, 2%, 2.2%, 2.5%, 2.8% or 3%, etc.

The mass percentage content of magnesium sulfate in the secondary seed culture medium is in a range from 0.8% to 2.4%, for example, it may be 1%, 1.2%, 1.5%, 1.8%, 2% or 2.2%, etc.

The mass percentage content of thiamine hydrochloride in the secondary seed culture medium is in a range from 0.2% to 0.8%, for example, it may be 0.3%, 0.4%, 0.5%, 0.6% or 0.7%, etc.

Preferably, the secondary seed culture medium further comprises a first trace element additive, and the usage amount of the first trace element additive is in a range from 0.8% to 2.6%, for example, it may be 1%, 1.2%, 1.5%, 1.8%, 2%, 2.2% or 2.5%, etc. In the present application, the percentages used in the description of the usage amount of the first trace element additive are mass percentages.

Preferably, the remainder in the secondary seed culture medium is water.

Preferably, the first trace element additive includes the following components: 4-8 g/L of copper sulfate, 0.12-0.16 g/L of sodium iodide, 5-6 g/L of manganese sulfate, 0.1-0.2 g/L of sodium molybdate, 0.04-0.08 g/L of boric acid, 0.6-0.8 g/L of cobalt chloride, 18-21 g/L of zinc chloride, 38-41 g/L of ferrous sulfate, 0.3-0.5 g/L of biotin and 3-5.2 g/L of sulfuric acid.

Specifically, the mass concentration of copper sulfate in the first trace element additive is in a range from 4 g/L to 8 g/L, for example, it may be 4.5 g/L, 5 g/L, 5.5 g/L, 6 g/L, 6.5 g/L, 7 g/L or 7.5 g/L, etc.

The mass concentration of sodium iodide in the first trace element additive is in a range from 0.12 g/L to 0.16 g/L, for example, it may be 0.125 g/L, 0.13 g/L, 0.135 g/L, 0.14 g/L, 0.145 g/L, 0.15 g/L or 0.155 g/L, etc.

The mass concentration of manganese sulfate in the first trace element additive is in a range from 5 g/L to 6 g/L, for example, it may be 5.1 g/L, 5.2 g/L, 5.3 g/L, 5.4 g/L, 5.5 g/L, 5.6 g/L, 5.7 g/L, 5.8 g/L or 5.9 g/L, etc.

The mass concentration of sodium molybdate in the first trace element additive is in a range from 0.1 g/L to 0.2 g/L, for example, it may be 0.11 g/L, 0.12 g/L, 0.13 g/L, 0.14 g/L, 0.15 g/L, 0.16 g/L, 0.17 g/L, 0.18 g/L or 0.19 g/L, etc.

The mass concentration of boric acid in the first trace element additive is in a range from 0.04 g/L to 0.08 g/L, for example, it may be 0.045 g/L, 0.05 g/L, 0.055 g/L, 0.06 g/L, 0.065 g/L, 0.07 g/L or 0.075 g/L, etc.

The mass concentration of cobalt chloride in the first trace element additive is in a range from 0.6 g/L to 0.8 g/L, for example, it may be 0.62 g/L, 0.65 g/L, 0.68 g/L, 0.7 g/L, 0.72 g/L, 0.75 g/L or 0.78 g/L, etc.

The mass concentration of zinc chloride in the first trace element additive is in a range from 18 g/L to 21 g/L, for example, it may be 18.5 g/L, 19 g/L, 19.5 g/L, 20 g/L or 20.5 g/L, etc.

The mass concentration of ferrous sulfate in the first trace element additive is in a range from 38 g/L to 41 g/L, for example, it may be 38.5 g/L, 39 g/L, 39.5 g/L, 40 g/L or 40.5 g/L, etc.

The mass concentration of biotin in the first trace element additive is in a range from 0.3 g/L to 0.5 g/L, for example, it may be 0.32 g/L, 0.35 g/L, 0.38 g/L, 0.4 g/L, 0.42 g/L, 0.45 g/L or 0.48 g/L, etc.

The mass concentration of sulfuric acid in the first trace element additive is in a range from 3 g/L to 5.2 g/L, for example, it may be 3.2 g/L, 3.5 g/L, 3.8 g/L, 4 g/L, 4.2 g/L, 4.5 g/L, 4.8 g/L or 5 g/L, etc.

Preferably, the primary seeds obtained from the primary seed culture are inoculated into a secondary seed culture medium at an inoculum size of 5% to 10% for secondary seed culture. The inoculum size of the primary seeds may be 6%, 7%, 8% or 9%, etc., further preferably 7% to 8%.

It should be noted that the term "inoculum size" refers to the ratio of the volume of the seed liquid transferred to the volume of the culture system after inoculation. Illustratively, an inoculum size of 5%-10% means that the volume fraction of the seed liquid is 5%-10%. In the following descriptions involving "inoculum size", it refers to the volume fraction, and for brevity, it will not be elaborated on individually.

Preferably, air (compressed air) is introduced during the secondary seed culture process.

Preferably, the aeration rate for the secondary seed culture is in a range from 0.1 vvm to 1.3 vvm, for example, it may be 0.2 vvm, 0.3 vvm, 0.5 vvm, 0.7 vvm, 0.9 vvm, 1 vvm or 1.2 vvm, etc.

Preferably, the secondary seed culture is carried out under stirring conditions, with the stirring speed ranging from 150 rpm to 900 rpm, for example, it may be 180 rpm, 200 rpm, 300 rpm, 400 rpm, 500 rpm, 600 rpm, 700 rpm or 800 rpm, etc.

As the preferred technical solution of the present application, during the secondary seed culture process, the stirring speed and the aeration rate of compressed air are gradually increased according to the continuous decrease of dissolved oxygen.

Illustratively, during the secondary seed culture process, when the initial dissolved oxygen is 100%, the stirring speed is 160-200 rpm, and the aeration rate is 0.1-0.3 vvm; when 80% ≤ dissolved oxygen < 100%, the stirring speed is 210-230 rpm, and the aeration rate is 0.4-0.5 vvm; when 60% ≤ dissolved oxygen < 80%, the stirring speed is 260-300 rpm, and the aeration rate is 0.3-0.5 vvm; when 40% ≤ dissolved oxygen < 60%, the stirring speed is 300-400 rpm, and the aeration rate is 0.4-0.6 vvm; when 20% ≤ dissolved oxygen < 40%, the stirring speed is 400-500 rpm, and the aeration rate is 0.5-0.7 vvm; when 10% ≤ dissolved oxygen < 20%, the stirring speed is 500-550 rpm, and the aeration rate is 0.6-0.8 vvm; when 0 < dissolved oxygen < 10%, the stirring speed is 550-650 rpm, and the aeration rate is 0.7-0.9 vvm; when the dissolved oxygen is 0, the stirring speed is 700-800 rpm, and the aeration rate is 0.9-1.2 vvm.

Preferably, the temperature for the secondary seed culture is in a range from 30°C to 33°C, for example, it may be 30.2°C, 30.5°C, 30.8°C, 31°C, 31.2°C, 31.5°C, 31.8°C, 32°C, 32.2°C, 32.5°C or 32.8°C, etc., further preferably 30°C to 32°C.

Preferably, the duration of the secondary seed culture is in a range from 8 h to 12 h, for example, it may be 8.5 h, 9 h, 9.5 h, 10 h, 10.5 h, 11 h or 11.5 h, etc.

Preferably, the secondary seed culture is processed to an OD value of 12 to 16, such as an OD value of 12.5, 13, 13.5, 14, 14.5, 15 or 15.5, obtaining the seed liquid.

Preferably, the carbon source of the fermentation culture medium includes a combination of the second acetate and the ketone compound.

Preferably, the second acetate includes any one or a combination of at least two of sodium acetate, potassium acetate and ammonium acetate, preferably sodium acetate.

Preferably, the mass concentration of the second acetate in the fermentation culture medium is in a range from 5 g/L to 15 g/L, for example, it may be 6 g/L, 7 g/L, 8 g/L, 9 g/L, 10 g/L, 11 g/L, 12 g/L, 13 g/L or 14 g/L, etc.

Preferably, the mass percentage of ketone compounds in the fermentation culture medium is in a range from 0.1% to 0.6%, for example, it may be 0.2%, 0.3%, 0.4% or 0.5%. Specific content of ketone compounds have catalytic functions on the intermediate products of the acetic acid metabolic pathway of the thallus during high-density fermentation, which helps to enhance the growth rate of the thallus and shorten the fermentation period.

Preferably, the fermentation culture medium includes the following components: 5-15 g/L of second acetate, 1%-3% of glucose, 0.6%-2% of yeast powder, 1.5%-3.2% of casein peptone, 0.8%-1.2% of magnesium sulfate, 0.6%-1.4% of thiamine hydrochloride, 0.5%-1% of betaine, 3.5%-6.8% of ammonium sulfate, 5%-7% of potassium dihydrogen phosphate, 2%-4% of disodium hydrogen phosphate and 0.1%-0.6% of ketone compounds.

Specifically, the mass concentration of the second acetate (preferably sodium acetate) in the fermentation culture medium is in a range from 5 g/L to 15 g/L, for example, it may be 6 g/L, 7 g/L, 8 g/L, 9 g/L, 10 g/L, 11 g/L, 12 g/L, 13 g/L or 14 g/L, etc.

The mass percentage content of glucose (anhydrous glucose) in the fermentation culture medium is in a range from 1% to 3%, for example, it may be 1.2%, 1.5%, 1.8%, 2%, 2.2%, 2.5% or 2.8%, etc.

The mass percentage content of yeast powder in the fermentation culture medium is in a range from 0.6% to 2%, for example, it may be 0.8%, 1%, 1.2%, 1.5% or 1.8%, etc.

The mass percentage content of casein peptone in the fermentation culture medium is in a range from 1.5% to 3.2%, for example, it may be 1.6%, 1.8%, 2%, 2.2%, 2.5%, 2.8% or 3%, etc.

The mass percentage content of magnesium sulfate in the fermentation culture medium is in a range from 0.8% to 1.2%, for example, it may be 0.85%, 0.9%, 0.95%, 1%, 1.05%, 1.1% or 1.15%, etc.

The mass percentage content of thiamine hydrochloride in the fermentation culture medium is in a range from 0.6% to 1.4%, for example, it may be 0.7%, 0.8%, 0.9%, 1%, 1.1%, 1.2% or 1.3%, etc.

The mass percentage content of betaine in the fermentation culture medium is in a range from 0.5% to 1%, for example, it may be 0.6%, 0.7%, 0.8% or 0.9%, etc.

The mass percentage content of ammonium sulfate in the fermentation culture medium is in a range from 3.5% to 6.8%, for example, it may be 3.8%, 4%, 4.2%, 4.5%, 4.8%, 5%, 5.2%, 5.5%, 5.8%, 6%, 6.2% or 6.5%, etc.

The mass percentage content of potassium dihydrogen phosphate in the fermentation culture medium is in a range from 5% to 7%, for example, it may be 5.2%, 5.5%, 5.8%, 6%, 6.2%, 6.5% or 6.8%, etc.

The mass percentage content of disodium hydrogen phosphate in the fermentation culture medium is in a range from 2% to 4%, for example, it may be 2.2%, 2.5%, 2.8%, 3%, 3.2%, 3.5% or 3.8%, etc.

The mass percentage content of ketone compounds in the fermentation culture medium is in a range from 0.1% to 0.6%, for example, it may be 0.2%, 0.3%, 0.4% or 0.5%, etc.

Preferably, the ketone compounds are C3-C10 ketone compounds, for example, the ketone compounds may be C3, C4, C5, C6, C7, C8, C9 or C10 ketone compounds, including aliphatic ketones and/or alicyclic ketones.

Preferably, the ketone compound includes any one or a combination of at least two of acetone, methyl ethyl ketone, and cyclohexanone; further preferably acetone.

As the preferred technical solution of the present application, the fermentation culture medium contains 0.1% to 0.6% of acetone. Since acetone has catalytic functions on the intermediate products in the acetic acid metabolic pathway, the introduction of acetone can further enhance the growth rate of the thallus and shorten the fermentation period.

Preferably, in the fermentation culture medium, further includes a second trace element additive, and the usage amount of the second trace element additive is in a range from 0.8% to 2.6%, for example, it may be 1%, 1.2%, 1.5%, 1.8%, 2%, 2.2% or 2.5%, etc. In the present application, the percentages used in the description of the usage amount of the second trace element additive are mass percentages.

Preferably, the remainder in the fermentation culture medium is water.

Preferably, the second trace element additive includes the following components: 4-8 g/L of copper sulfate, 0.12-0.16 g/L of sodium iodide, 5-6 g/L of manganese sulfate, 0.1-0.2 g/L of sodium molybdate, 0.04-0.08 g/L of boric acid, 0.6-0.8 g/L of cobalt chloride, 18-21 g/L of zinc chloride, 38-41 g/L of ferrous sulfate, 0.3-0.5 g/L of biotin, and 3-5.2 g/L of sulfuric acid.

Specifically, the mass concentration of copper sulfate in the second trace element additive is in a range from 4 g/L to 8 g/L, for example, it may be 4.5 g/L, 5 g/L, 5.5 g/L, 6 g/L, 6.5 g/L, 7 g/L or 7.5 g/L, etc.

The mass concentration of sodium iodide in the second trace element additive is in a range from 0.12 g/L to 0.16 g/L, for example, it may be 0.125 g/L, 0.13 g/L, 0.135 g/L, 0.14 g/L, 0.145 g/L, 0.15 g/L or 0.155 g/L, etc.

The mass concentration of manganese sulfate in the second trace element additive is in a range from 5 g/L to 6 g/L, for example, it may be 5.1 g/L, 5.2 g/L, 5.3 g/L, 5.4 g/L, 5.5 g/L, 5.6 g/L, 5.7 g/L, 5.8 g/L or 5.9 g/L, etc.

The mass concentration of sodium molybdate in the second trace element additive is in a range from 0.1 g/L to 0.2 g/L, for example, it may be 0.11 g/L, 0.12 g/L, 0.13 g/L, 0.14 g/L, 0.15 g/L, 0.16 g/L, 0.17 g/L, 0.18 g/L or 0.19 g/L, etc.

The mass concentration of boric acid in the second trace element additive is in a range from 0.04 g/L to 0.08 g/L, for example, it may be 0.045 g/L, 0.05 g/L, 0.055 g/L, 0.06 g/L, 0.065 g/L, 0.07 g/L or 0.075 g/L, etc.

The mass concentration of cobalt chloride in the second trace element additive is in a range from 0.6 g/L to 0.8 g/L, for example, it may be 0.62 g/L, 0.65 g/L, 0.68 g/L, 0.7 g/L, 0.72 g/L, 0.75 g/L or 0.78 g/L, etc.

The mass concentration of zinc chloride in the second trace element additive is in a range from 18 g/L to 21 g/L, for example, it may be 18.5 g/L, 19 g/L, 19.5 g/L, 20 g/L or 20.5 g/L, etc.

The mass concentration of ferrous sulfate in the second trace element additive is in a range from 38 g/L to 41 g/L, for example, it may be 38.5 g/L, 39 g/L, 39.5 g/L, 40 g/L or 40.5 g/L, etc.

The mass concentration of biotin in the second trace element additive is in a range from 0.3 g/L to 0.5 g/L, for example, it may be 0.32 g/L, 0.35 g/L, 0.38 g/L, 0.4 g/L, 0.42 g/L, 0.45 g/L or 0.48 g/L, etc.

The mass concentration of sulfuric acid in the second trace element additive is in a range from 3 g/L to 5.2 g/L, for example, it may be 3.2 g/L, 3.5 g/L, 3.8 g/L, 4 g/L, 4.2 g/L, 4.5 g/L, 4.8 g/L or 5 g/L, etc.

Preferably, the seed liquid is inoculated into a fermentation culture medium at an inoculum size of 3% to 6% for high-density fermentation. The inoculum size of the seed liquid may be 3.5%, 4%, 4.5%, 5% or 5.5%, etc.

Preferably, the nitrogen source supplemented during the high-density fermentation process includes any one or a combination of at least two of urea, ammonium sulfate, ammonium nitrate and ammonia water; further preferably urea.

As the preferred technical solution of the present application, the carbon source supplemented during the high-density fermentation process is acetic acid, and the nitrogen source supplemented is urea. Since the optimal pH for the growth of thallus is a neutral environment, but alkaline substances are produced during the fermentation process, the pH value will gradually increases. Therefore, supplementing acetic acid can not only neutralize and stabilize the pH but also provide a carbon source for growth, and it can be directly added without sterilization. Moreover, acetic acid possesses a high carbon content and can be supplemented without the need for preparation into an aqueous solution, which is more conducive to achieving high-density fermentation. In contrast, if a carbon source that requires preparation into an aqueous solution is selected (such as glucose), supplementing it into the fermentation system would simultaneously introduce additional water. This increases the volume of the fermentation broth, leading to its dilution and making it difficult to achieve high-density fermentation. Using urea as a nitrogen source is advantageous as it is more readily absorbed by the thallus, thereby facilitating protein synthesis. through the design and compounding of the supplemented carbon source and nitrogen source, the rapid growth of thallus and synthesis of protein are promoted more effectively.

Preferably, the urea is supplemented in the form of an aqueous urea solution.

Preferably, the mass percentage content of urea in the aqueous urea solution is in a range from 30% to 50%, such as 32%, 35%, 38%, 40%, 42%, 45% or 48%, etc., further preferably 40%.

Preferably, the temperature for the high-density fermentation ranges from 27°C to 33°C, for example, it may be 27.5°C, 28°C, 28.5°C, 29°C, 29.5°C, 30°C, 30.5°C, 31°C, 31.5°C, 32°C or 32.5°C, etc., further preferably 28°C to 32°C.

Preferably, the pH value for the high-density fermentation ranges from 6.4 to 7.1, for example, it may be 6.5, 6.6, 6.7, 6.8, 6.9 or 7, etc., further preferably 6.5 to 7.

Preferably, the high-density fermentation is carried out under stirring conditions, with the stirring speed ranging from 130 rpm to 800 rpm, for example, it may be 150 rpm, 200 rpm, 300 rpm, 400 rpm, 500 rpm, 600 rpm, 700 rpm or 750 rpm, etc.

Preferably, the aeration rate for the high-density fermentation is 0.5 vvm to 2.4 vvm, for example, it may be 0.6 vvm, 0.8 vvm, 1 vvm, 1.2 vvm, 1.5 vvm, 1.8 vvm, 2 vvm or 2.2 vvm, etc.

Preferably, the gas introduced during the high-density fermentation process includes air and, optionally, oxygen (pure oxygen).

As the preferred technical solution of the present application, regulating the composition and flow rate of the gas introduced during the high-density fermentation process is more conducive to promoting the growth of the thallus and the synthesis of protein. Specifically:

when the OD value is less than 300, air (compressed air) is introduced, with an aeration rate of 0.6 vvm to 1 vvm; when $300 \leq$ OD value $< 500$, a mixed gas of air and oxygen is introduced, with a ratio of air to oxygen of (1.5-2.5):1, for example, the ratio may be 1.6:1, 1.8:1, 2:1, 2.2:1, 2.4:1, etc., preferably 2:1; the aeration rate is 0.8 vvm to 1.2 vvm; when the OD value is in a range from 500 to 700, a mixed gas of air and oxygen is introduced, with a ratio of air to oxygen of (0.8-1.2):1, for example, the ratio may be 0.9:1, 1:1, 1.1:1, etc., preferably 1:1; the aeration rate is 1.2 vvm to 1.8 vvm; when the OD value is greater than 700, a mixed gas of air and oxygen is introduced, with a ratio of air to oxygen of 1: (1.5-2.5), for example, the ratio may be 1:1.6, 1:1.8, 1:2, 1:2.2 or 1:2.4, etc., preferably 1:2; the aeration rate is 1.8 vvm to 2.4 vvm.

Preferably, the method for determining the endpoint of high-density fermentation is as follows: monitor the OD value of the high-density fermentation system. If the increase in OD value per hour is less than or equal to 20 within two consecutive hours, indicating slow growth of the thallus, it is judged that no further growth will occur, and the endpoint of high-density fermentation is reached. The fermentation broth is then released from the fermentation tank.

In the present application, "OD value" refers to the OD value of the fermentation broth (culture system), which is obtained through ultraviolet spectrophotometry, specifically the OD value at 600 nm.

Preferably, the high-density fermentation is processed to an OD value 780-820, such as an OD value of 782, 785, 788, 790, 792, 795, 798, 800, 802, 805, 808, 810, 812, 815 or 818, etc., and the fermentation is terminated to obtain the single-cell protein.

Preferably, the high-density fermentation includes a first fermentation stage, a second fermentation stage, and a third fermentation stage, with the OD values increasing sequentially.

The OD value of the first fermentation stage is less than 500, and the carbon to nitrogen ratio of the supplemented acetic acid and nitrogen source in the first fermentation stage is greater than 18, such as a carbon to nitrogen ratio of 20, 25, 30, 35, 40, 45, 50, 55, 60, 65, 70, 75, 80, 85, 90 or 100, etc.

The OD value of the second fermentation stage is in a range from 500 to 700, and the carbon to nitrogen ratio of the supplemented acetic acid and nitrogen source in the second fermentation stage is in a range from 5 to 18, such as a carbon to nitrogen ratio of 6, 7, 8, 9, 10, 11, 12, 13, 14, 15, 16 or 17, etc.

The OD value of the third fermentation stage is greater than 700, and the carbon to nitrogen ratio of the supplemented acetic acid and nitrogen source in the third fermentation stage is less than 5, such as a carbon to nitrogen ratio of 1, 1.5, 2, 2.5, 3, 3.5, 4 or 4.5, etc.

As the preferred technical solution of the present application, the carbon to nitrogen ratio of the carbon sources and nitrogen sources supplemented during the high-density fermentation process is regulated. In the early stage with a relatively low OD value, the carbon to nitrogen ratio is set to be greater than 18, which is conducive to vigorous growth of the thallus. When the OD value is 500-700, the carbon to nitrogen ratio is set to be 5-18, which is beneficial for both thallus growth and protein synthesis simultaneously. In the later stage of high-density fermentation, the carbon to nitrogen ratio is set to be less than 5, slowing down thallus growth while increasing the rate of protein synthesis. This fully synthesizes proteins and is conducive to improving the content of crude protein in the fermentation product. The present application can achieve the fastest growth of thallus and the highest crude protein content by adjusting the carbon to nitrogen ratio, which shortens the fermentation culture period while increasing the yield of protein, thereby simultaneously improving the production efficiency and yield of the single-cell protein.

In the present application, the feeding rate of supplemented acetic acid and nitrogen source can be regulated according to the carbon to nitrogen ratio at different stages of high-density fermentation; illustratively, the feeding rate of acetic acid is 0.6 g/L to 2.8 g/L, for example, it may be 0.8 g/L, 1 g/L, 1.2 g/L, 1.5 g/L, 1.8 g/L, 2 g/L, 2.2 g/L, 2.5 g/L or 2.7 g/L, etc., further preferably 0.8 g/L to 2.5 g/L; the feeding rate of the nitrogen source is 0.1 g/L to 3.2 g/L, for example, it may be 0.2 g/L, 0.5 g/L, 0.8 g/L, 1 g/L, 1.2 g/L, 1.5 g/L, 1.8 g/L, 2 g/L, 2.2 g/L, 2.5 g/L, 2.8 g/L or 3 g/L, etc.

Preferably, during the initial stage of high-density fermentation, the feeding rates of acetic acid and nitrogen sources are regulated, the carbon to nitrogen ratio is set to be greater than 18 to promote vigorous growth of the thallus. As the fermentation progresses, the feeding rates of acetic acid and nitrogen sources are adjusted to maintain a carbon to nitrogen ratio of 5-18, facilitating simultaneous rapid growth of the thallus and rapid protein synthesis. Further adjustments to the feeding rates of acetic acid and nitrogen sources are made to maintain a carbon to nitrogen ratio of less than 5, thereby facilitating the synthesis of single-cell protein and increasing the content of crude protein. Throughout the high-density fermentation stage, the optimal effect of achieving the fastest thallus growth rate and highest crude protein content is achieved by regulating and balancing the carbon to nitrogen ratio.

As the preferred technical solution of the present application, by regulating the processes during different stages of high-density fermentation, including pH range, temperature, rotation speed, carbon to nitrogen ratio, dissolved oxygen and gas flow rate, the growth environment of the thallus is adjusted to achieve optimal growth conditions for the thallus and the best state for single-cell protein synthesis.

Preferably, the first fermentation stage includes a stage A, a stage B, and a stage C carried out sequentially, whereby the high-density fermentation includes a stage A, a stage B, a stage C, a second fermentation stage, and a third fermentation stage carried out sequentially, specifically as follows:

in the stage A, the OD value is less than 100, with a temperature ranging from 31°C to 33°C, a pH value of 6.4 to 6.6, an aeration rate of 0.5 vvm to 0.7 vvm, a rotation speed of 130 rpm to 170 rpm, and a carbon to nitrogen ratio of greater than 18 for the supplemented acetic acid and nitrogen source;
further preferably, in the stage A, air (compressed air) is introduced, with a controlled temperature of 32°C, a pH value of 6.5, an aeration rate of 0.6 vvm, a rotation speed of 150 rpm, an acetic acid feeding rate of 0.8 L/h, and a controlled carbon to nitrogen ratio of greater than 18. Under these conditions, the thallus grow rapidly.
in the stage B, 100 ≤ OD value < 300, with a temperature ranging from 30°C to 32°C, a pH value of 6.5 to 6.7, an aeration rate of 0.9 vvm to 1.1 vvm, a rotation speed of 180 rpm to 220 rpm, and a carbon to nitrogen ratio of greater than 18 for the supplemented acetic acid and nitrogen source;
further preferably, in the stage B, air (compressed air) is introduced, with a controlled temperature of 31°C, a pH of 6.6, an aeration rate of 1 vvm, a rotation speed of 200 rpm, an acetic acid feeding rate of 1.2 L/h, and a controlled carbon to nitrogen ratio of greater than 18. Under these conditions, the thallus grow rapidly.
in the stage C, 300 ≤ OD value < 500, with a temperature ranging from 29°C to 31°C, a pH value of 6.6 to 6.8, an aeration rate of 1.1 vvm to 1.3 vvm, a rotation speed of 280 rpm to 320 rpm, and a carbon to nitrogen ratio of greater than 18 for the supplemented acetic acid and nitrogen source;
further preferably, in the stage C, a mixed gas of air and oxygen is introduced, with a ratio of air to oxygen of (1.5-2.5):1, more preferably 2:1. The temperature is controlled at 30°C, the pH value at 6.7, the aeration rate at 1.2 vvm, the rotation speed at 300 rpm, the acetic acid feeding rate at 1.6 L/h, and the carbon to nitrogen ratio is controlled to be greater than 18. Under these conditions, the thallus grow rapidly.

The OD value of the second fermentation stage is in a range from 500 to 700, with a temperature ranging from 28°C to 30°C, a pH value of 6.7 to 6.9, an aeration rate of 1.5 vvm to 1.7 vvm, a rotation speed of 450 rpm to 550 rpm, and a carbon to nitrogen ratio of 5 to 18 for the supplemented acetic acid and nitrogen source;
further preferably, in the second fermentation stage, a mixed gas of air and oxygen is introduced, with a ratio of air to oxygen of (0.8-1.2):1, more preferably 1:1. The temperature is controlled at 29°C, the pH value at 6.8, the aeration rate at 1.6 vvm, the rotation speed at 500 rpm, the acetic acid feeding rate at 2.2 L/h, and the carbon to nitrogen ratio is controlled at 5-18. Under these conditions, the growth rate of the thallus is normal, and single-cell protein is synthesized simultaneously.

The OD value of the third fermentation stage is greater than 700, with a temperature ranging from 27°C to 29°C, a pH value of 6.9 to 7.1, an aeration rate of 1.9 vvm to 2.1 vvm, a rotation speed of 650 rpm to 750 rpm, and a carbon to nitrogen ratio of less than 5 for the supplemented acetic acid and nitrogen source;
further preferably, in the third fermentation stage, a mixed gas of air and oxygen is introduced, with a ratio of air to oxygen of 1:(1.5-2.5), more preferably 1:2. The temperature is controlled at 28°C, the pH value at 7, the aeration rate at 2.0 vvm, the rotation speed at 700 rpm, the acetic acid feeding rate at 2.5 L/h, and the carbon to nitrogen ratio is controlled to be less than 5. Under these conditions, the growth rate of the thallus is slow, allowing for sufficient synthesis of single-cell protein.

When the OD value reaches 780-820 and the thallus growth is slow, the fermentation can be completed by releasing the fermentation tank to obtain the fermentation broth.

Preferably, after the completion of the high-density fermentation, the method further includes a post-treatment step.

Preferably, the method of the post-treatment includes: subjecting the fermentation broth obtained from the high-density fermentation to solid-liquid separation, washing, and drying in sequence to obtain the single-cell protein.

Preferably, the method of the solid-liquid separation is centrifugal separation.

Preferably, the detergent used for the washing is water.

Preferably, the drying method includes spray drying.

Preferably, the method specifically includes the following steps:

(1) inoculating the Yarrowia lipolytica strain into the primary seed culture medium, and culturing for 16 h to 18 h at a temperature of 30°C to 33°C and a rotation speed of 100 rpm to 300 rpm until the OD value reaches 4.2 to 6.8, thereby obtaining the primary seed;

the primary seed culture medium includes the following components: 5-15 g/L of sodium acetate, 1%-3% of glucose, 0.6%-2% of yeast powder and 1.5%-3.2% of casein peptone;

(2) inoculating the primary seed into the secondary seed culture medium at an inoculum size of 5% to 10%, and culturing for 8 h to 12 h at a temperature of 30°C to 33°C, a rotation speed of 150 rpm to 900 rpm and an aeration rate of 0.1 vvm to 1.3 vvm until the OD value reaches 12 to 16, thereby obtaining the secondary seed, i.e., the seed liquid;

the secondary seed culture medium includes the following components: 5-20 g/L of sodium acetate, 1%-3% of glucose, 0.6%-2% of yeast powder, 1.5%-3.2% of casein peptone, 0.8%-2.4% of magnesium sulfate, 0.2%-0.8% of thiamine hydrochloride, and 0.8%-2.6% of first trace element additive;

the first trace element additive includes the following components: 4-8 g/L of copper sulfate, 0.12-0.16 g/L of sodium iodide, 5-6 g/L of manganese sulfate, 0.1-0.2 g/L of sodium molybdate, 0.04-0.08 g/L of boric acid, 0.6-0.8 g/L of cobalt chloride, 18-21 g/L of zinc chloride, 38-41 g/L of ferrous sulfate, 0.3-0.5 g/L of biotin, and 3-5.2 g/L of sulfuric acid;

(3) inoculate the seed liquid into the fermentation culture medium at an inoculum size of 3% to 6% for high-density fermentation., and supplement with acetic acid and urea during the process of high-density fermentation until the OD value reaches 780 to 820, thereby obtaining the single-cell protein;

the fermentation culture medium includes the following components: 5-15 g/L of sodium acetate, 1%-3% of glucose, 0.6%-2% of yeast powder, 1.5%-3.2% of casein peptone, 0.8%-1.2% of magnesium sulfate, 0.6%-1.4% of thiamine hydrochloride, 0.5%-1% of betaine, 3.5%-6.8% of ammonium sulfate, 5%-7% of potassium dihydrogen phosphate, 2%-4% of disodium hydrogen phosphate, 0.1%-0.6% of acetone, and 0.8%-2.6% of second trace element additives;

the second trace element additive includes the following components: 4-8 g/L of copper sulfate, 0.12-0.16 g/L of sodium iodide, 5-6 g/L of manganese sulfate, 0.1-0.2 g/L of sodium molybdate, 0.04-0.08 g/L of boric acid, 0.6-0.8 g/L of cobalt chloride, 18-21 g/L of zinc chloride, 38-41 g/L of ferrous sulfate, 0.3-0.5 g/L of biotin, and 3-5.2 g/L of sulfuric acid;

the high-density fermentation includes a first fermentation stage, a second fermentation stage, and a third fermentation stage, with the OD value increasing sequentially;

the OD value of the first fermentation stage is less than 500, with a temperature ranging from 29°C to 33°C, a pH value of 6.5 to 6.8, an aeration rate of 0.5 vvm to 1.3 vvm, a rotation speed of 280 rpm to 320 rpm, and a carbon to nitrogen ratio of greater than 18 for the supplemented acetic acid and urea;

the OD value of the second fermentation stage is in a range from 500 to 700, with a temperature ranging from 28°C to 30°C, a pH value of 6.7 to 6.9, an aeration rate of 1.5 vvm to 1.7 vvm, a rotation speed of 450 rpm to 550 rpm, and a carbon to nitrogen ratio of 5 to 18 for the supplemented acetic acid and nitrogen source;

the OD value of the third fermentation stage is greater than 700, with a temperature ranging from 27°C to 29°C, a pH value of 6.9 to 7.1, an aeration rate of 1.9 vvm to 2.1 vvm, a rotation speed of 650 rpm to 750 rpm, and a carbon to nitrogen ratio of less than 5 for the supplemented acetic acid and nitrogen source.

As the preferred technical solution of the present application, the duration for the high-density fermentation (i.e., fermentation period) is in a range from 25 h to 60 h, for example, it may be 26 h, 28 h, 30 h, 32 h, 35 h, 38 h, 40 h, 42 h, 45 h, 48 h, 50 h, 52 h, 55 h, 58 h, etc. Further preferably 28-36 h, and more preferably 28-32 h.

Preferably, the total yield (dry weight) of the single-cell protein is greater than 150 g/L, for example, it may be 155 g/L, 160 g/L, 170 g/L, 180 g/L, 190 g/L, 200 g/L, 210 g/L, 220 g/L, 230 g/L, 240 g/L, 250 g/L or 260 g/L, etc. Further preferably, greater than or equal to 160 g/L, and even more preferably, 200-250 g/L.

Preferably, the yield of the single-cell protein is greater than 2.5 g/(·h), for example, it may be 2.6 g/(L·h), 2.8 g/(L·h), 3 g/(L·h), 3.5 g/(L·h), 4 g/(·h), 5 g/(L·h), 6 g/(·h), 7 g/(·h), 8 g/(·h), 8.5 g/(L·h), 9 g/(L·h) or 10 g/(·h), etc. Further preferably, greater than or equal to 3 g/(L·h), and even more preferably, 6-9 g/(L·h).

Preferably, the mass percentage content of crude protein in the single-cell protein is greater than or equal to 35%, for example, it may be 38%, 40%, 42%, 45%, 48%, 50%, 52%, 55%, 58%, 60%, 62%, 65% or 68%, etc. Further preferably, greater than or equal to 55%, and even more preferably, greater than or equal to 60%.

Compared to related technologies, the examples of the present application have the following beneficial effects:

(1) In the method for producing single-cell protein by means of high-density fermentation provided by the embodiments of the present application, by introducing the first acetate into the carbon source of the seed culture medium, the formation of a unique acetic acid metabolic pathway of thallus is induced and promoted, thereby laying the foundation for the subsequent supplementation of acetic acid as a carbon source and promoting rapid growth of the thallus during high-density fermentation. Meanwhile, the fermentation culture medium contains a second acetate and/or ketone compounds, which promote rapid growth of thallus. During the high-density fermentation stage, nitrogen sources and acetate are supplemented to maintain the thallus in an optimal neutral pH growth environment, and the carbon to nitrogen ratio is regulated to supply the thallus with appropriate nutrients for their continuous growth, effectively enhancing the growth rate of the thallus and the synthesis rate of single-cell protein. Through the compounding of specific materials and processes, the method can realize high-density fermentation of thallus and efficient synthesis of single-cell protein in a short period of time, a fermentation product with a higher content of crude proteins can be obtained while the culture time of high-density fermentation is significantly shortened, and the production efficiency and yield of single-cell proteins are greatly improved.

(2) Through further optimization and design of material and process, adaptive medium composition, specific feeding methods, and stepwise process regulation strategies during high-density fermentation, the examples of the present application achieve rapid growth of the thallus and rapid synthesis of single-cell protein. This results in a high-density fermentation time (fermentation period) of 28-30 h, which is shortened to one-third compared to the production duration of single-cell protein in related technologies. Meanwhile, the dry weight of single-cell protein is 200-243 g/L, and the yield is greater than or equal to 6 g/(·h), which can increase by 20 times compared to the yield of the same type. The content of crude protein is greater than or equal to 60%, which can be increased by 50% compared to related technologies, thus significantly improving the yield and production capacity of single-cell protein.

After reading and comprehending the accompanying drawings and detailed descriptions, other aspects can be understood.

## DESCRIPTION OF THE DRAWINGS

The accompanying drawings are used to facilitate further understanding of the technical solutions presented in the present application and constitute a part of the specification. They are used together with the embodiments of the present application to explain the technical solution presented in the present application and do not constitute limitations on the technical solution of the present application.

Figure 1 is a growth trend graph of the high-density fermentation process in the method provided in Example 1;
Figure 2 is a test diagram of the fermentation broth obtained in the method provided in Example 1.

## DETAILED DESCRIPTION

The technical solution of the present application will be further explained through specific embodiments. Those skilled in the art should understand that the described examples are merely intended to assist in understanding the present application and should not be considered as specific limitations on the present application.

The terms "comprise", "include", "have", "contain", or any other variations thereof used in this article are intended to cover non-exclusive inclusion. For example, a composition, step, method, product or device that comprises the listed elements is not necessarily limited to those elements, but may also include other elements not explicitly listed or the inherent elements to such composition, step, method, product or device.

In the present application, features designated as "first" and "second" may explicitly or implicitly include one or more of such features, used to distinguish and describe the features, without any distinction of order or importance. In the description of the present application, unless otherwise stated, the meaning of "multiple" is two or more than two.

In the present application, the term "OD value" refers to the OD value of the fermentation broth, which is obtained through ultraviolet spectrophotometry. Specifically, an ultraviolet spectrophotometer (Alpha-1106, Shanghai lab-spectrum Instrument Co., Ltd.) is used to measure the OD value of the fermentation broth at 600 nm.

In the following specific embodiments of the present application, the testing methods for dry weight, yield, and crude protein content are as follows:

(1) Dry weight of single-cell protein: the unit is g/L

1.00 L of fermentation broth is weighed and centrifuged at 8000 revolutions per minute (rpm) for 10 min. After the thallus are obtained, 1 L of water is then added for uniform resuspension. The mixture is centrifuged again at 8,000 rpm for 10 minutes to obtain wet thallus, the mass of which is weighed and recorded as $m_1$, 5.000 g of wet thallus is taken from $m_1$ and dried at $103 \pm 2°C$ according to the method described in the national standard GB/T 6435-2014 "Determination of Moisture in Feeds" to obtain dry thallus, the mass of which is weighed as $m_2$ (accurate to 4 decimal places).

$$\text{Dry cell weight (DCW)} = m_2/5.000 \times m_1/1.00$$

(2) Yield: the unit is g/(L·h)

$$\text{Yield} = \text{Dry weight of single-cell protein} \div \text{Duration of high-density fermentation (fermentation period)}.$$

(3) Crude protein content

According to the national standard GB/T 6432-2018 "Determination of Crude Protein in Feeds - Kjeldahl Method", the crude protein content (mass fraction) is obtained through digestion, ammonia distillation and titration.

In the following specific embodiments of the present application, the Yarrowia lipolytica strain was purchased from the China Center of Industrial Culture Collection, with the accession number CICC 32291; various components used for preparing the culture medium were commercially available chemicals.

In the following specific embodiments of the present application, the method for determining the endpoint of high-density fermentation is as follows: if the increase in OD value per hour is less than 20 within two consecutive hours, indicating very slow growth of the thallus, it is judged that no further growth will occur, and the endpoint of high-density fermentation is reached. The fermentation broth is then released from the fermentation tank.

Example 1

A method for producing single-cell protein by means of high-density fermentation, specifically comprising the following steps:

(1) Primary seed culture:
200 mL of primary seed culture medium was prepared, comprising the following components: 8 g/L of sodium acetate, 1.8% of anhydrous glucose, 1.2% of yeast powder, and 2.2% of casein peptone; the primary seed culture medium was sterilized at 121°C for 20 min in a sterilizer. After sterilization, the primary seed culture medium was placed in a super clean bench. After returning to room temperature, the thawed Yarrowia lipolytica strain cryopreservation solution was added to the primary seed culture medium and placed in a shaker, and then cultivated for 16 h at a temperature of 32°C and a rotation speed of 180 rpm, with an OD value of about 4.2, whereby the primary seed was obtained;
(2) Secondary seed culture:

2L of secondary seed culture medium was prepared, comprising the following components: 15g/L of sodium acetate, 2.2% of anhydrous glucose, 1.5% of yeast powder, 2.5% of casein peptone, 1.2% of magnesium sulfate, and 0.6% of thiamine hydrochloride;
the secondary seed culture medium was added to a 5L fermentation tank and sterilized at 121°C for 20 min in a sterilizer, after cooling to 32°C, the primary seed was added to the secondary seed culture medium fermentation tank at an inoculum size of 7.5% through flame inoculation;
20 mL of trace element additive after sterilization and filtration was then added, with the following formula: 4 g/L of copper sulfate, 0.12 g/L of sodium iodide, 5 g/L of manganese sulfate, 0.1 g/L of sodium molybdate, 0.04 g/L of boric acid, 0.6 g/L of cobalt chloride, 18 g/L of zinc chloride, 38 g/L of ferrous sulfate, 0.3 g/L of biotin, and 3 g/L of sulfuric acid.

In the secondary seed culture, when the initial dissolved oxygen was at 100%, the stirring speed was set to 180 rpm, the temperature was 32°C, and the aeration rate of compressed air was 0.2 vvm; when the dissolved oxygen ranged from 80% to 100%, the stirring speed was set to 220 rpm, the temperature was 32°C, and the aeration rate of compressed air was 0.3 vvm; when the dissolved oxygen ranged from 60% to 80%, the stirring speed was set to 280 rpm, the temperature was 32°C, and the aeration rate of compressed air was 0.4 vvm; when the dissolved oxygen ranged from 40% to 60%, the stirring speed was set to 350 rpm, the temperature was 32°C, and the aeration rate of compressed air was 0.5 vvm; when the dissolved oxygen ranged from 20% to 40%, the stirring speed was set to 440 rpm, the temperature was 31°C, and the aeration rate of compressed air was 0.6 vvm; when the dissolved oxygen ranged from 10% to 20%, the stirring speed was set to 520 rpm, the temperature was 31°C, and the aeration rate of compressed air was 0.7 vvm; when the dissolved oxygen ranged from 0% to 10%, the stirring speed was set to 600 rpm, the temperature was 30°C, and the aeration rate of compressed air was 0.8 vvm; when the dissolved oxygen was at 0%, the stirring speed was set to 700 rpm, the temperature was 30°C, and the aeration rate of compressed air was 0.9 vvm.

The duration for the secondary seed culture was 8 h, with an OD value of about 12, resulting in the secondary seed.

(3) High-density fermentation

40 L of fermentation culture medium was prepared, comprising the following components: 15 g/L of sodium acetate, 2.6% of anhydrous glucose, 1.8% of yeast powder, 2.8% of casein peptone, 1.2% of magnesium sulfate, 1.4% of thiamine hydrochloride, 1% of betaine, 6.8% of ammonium sulfate, 7% of potassium dihydrogen phosphate, 4% of disodium hydrogen phosphate, and 0.2% of acetone;

the fermentation culture medium was added to a 100 L fermentation tank and sterilized with steam through the fermentation tank, after cooling to 32°C, the secondary seed was added to the 100 L fermentation tank at an inoculum size of 5%;

400 mL of trace elements after sterilization and filtration were then added to the culture medium in the fermentation tank, with the following formula: 8 g/L of copper sulfate, 0.16 g/L of sodium iodide, 6 g/L of manganese sulfate, 0.2 g/L of sodium molybdate, 0.08 g/L of boric acid, 0.8 g/L of cobalt chloride, 21 g/L of zinc chloride, 41 g/L of ferrous sulfate, 0.45 g/L of biotin, and 5.2 g/L of sulfuric acid.

The supplemented carbon source and nitrogen source were prepared. The carbon source was acetic acid, and the nitrogen source was a sterilized 40% urea solution.

In high-density fermentation, the growth environment of the thallus was adjusted to achieve optimal conditions for thallus growth and single-cell protein synthesis by regulating the pH range, temperature, rotation speed, carbon to nitrogen ratio, dissolved oxygen, and gas flow rate during different stages of the process. The specifics were as follows:

under the conditions of $0 \leq$ OD value <100, a controlled temperature of 32°C, a pH of 6.5, an aeration rate of compressed air of 0.6 vvm, a rotation speed of 150 rpm, a carbon source feeding rate of 0.8 L/h, and a controlled carbon to nitrogen ratio of 20, the thallus grew rapidly;

under the conditions of $100 \leq$ OD value < 300, a controlled temperature of 31°C, a pH of 6.6, an aeration rate of compressed air of 1 vvm, a rotation speed of 200 rpm, a carbon source feeding rate of 1.2 L/h, and a controlled carbon to nitrogen ratio of 20, the thallus grew rapidly;

under the conditions of $300 \leq$ OD value < 500, a controlled temperature of 30°C, a pH of 6.7, an aeration rate of a mixed gas (air: pure oxygen at a ratio of 2:1) of 1.2 vvm, a rotation speed of 300 rpm, a carbon source feeding rate of 1.6 L/h, and a controlled carbon to nitrogen ratio of 20, the thallus grew rapidly;

under the conditions of an OD value of 500 to 700, a controlled temperature of 29°C, a pH of 6.8, an aeration rate of a mixed gas (air: pure oxygen at a ratio of 1:1) of 1.6 vvm, a rotation speed of 500 rpm, a carbon source feeding rate of 2.2 L/h, and a controlled carbon to nitrogen ratio of 10, the growth rate of the thallus was normal, and single-cell protein was synthesized simultaneously;

under the conditions of an OD value greater than 700, a controlled temperature of 28°C, a pH of 7, an aeration rate of a mixed gas (air: pure oxygen at a ratio of 1:2) of 2.0 vvm, a rotation speed of 700 rpm, a carbon source feeding rate of 2.5 L/h, and a controlled carbon to nitrogen ratio of 3, the growth rate of the thallus was slow, allowing for sufficient synthesis of single-cell protein.

After 30 h of cultivation, the OD value reached 790, indicating slow growth of the thallus. The fermentation broth was then released from the fermentation tank.

(4) Post-treatment

The fermentation broth was centrifuged and washed with water, then spray dried to obtain single-cell protein with a dry weight of 240 g/L, a yield of 8 g/(L·h), and a crude protein content of 62%.

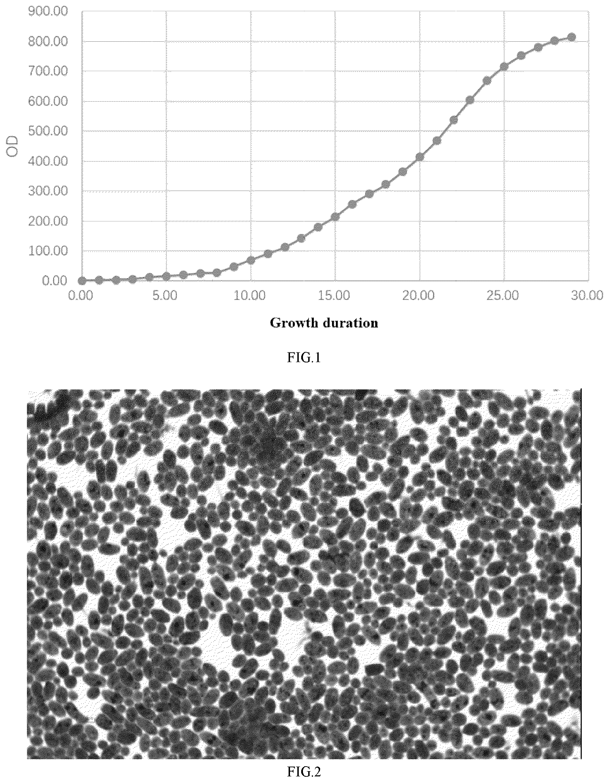

During the high-density fermentation process of this example, samples were taken every hour. Based on the absorption peak of the OD value of the fermentation broth at 600 nm, the thallus OD was calculated after conversion and correction. The average value was obtained from three measurements. A growth trend graph was plotted based on growth time and OD value. The graph obtained is shown in Figure 1.

A sample of the fermentation broth obtained from high-density fermentation in this example was taken for microscopic examination. The testing instrument was a digital microscope (longbase 610D, Qingdao Longbase Medical Equipment Co., Ltd.). The test image obtained is shown in Figure 2.

Example 2

A method for producing single-cell protein by means of high-density fermentation, specifically comprising the following steps:

(1) Primary seed culture:
200 mL of primary seed culture medium was prepared, comprising the following components: 8 g/L of sodium acetate, 1.8% of anhydrous glucose, 1.2% of yeast powder, and 2.2% of casein peptone; the primary seed culture medium was sterilized at 121°C for 20 min in a sterilizer. After sterilization, the primary seed culture medium was placed in a super clean bench. After returning to room temperature, the thawed Yarrowia lipolytica strain cryopreservation solution was added to the primary seed culture medium and placed in a shaker, and then cultivated for 17 h at a temperature of 32°C and a rotation speed of 180 rpm, with an OD value of about 4.8, whereby the primary seed was obtained;

(2) Secondary seed culture:

2L of secondary seed culture medium was prepared, comprising the following components: 15g/L of sodium acetate, 2.2% of anhydrous glucose, 1.5% of yeast powder, 2.5% of casein peptone, 1.2% of magnesium sulfate, and 0.6% of thiamine hydrochloride;
the secondary seed culture medium was added to a 5L fermentation tank, and sterilized at 121°C for 20 min in a sterilizer, after cooling to 32°C, the primary seed was added to the secondary seed culture medium fermentation tank at an inoculum size of 7.5% through flame inoculation;
20 mL of trace element additive after sterilization and filtration was then added, with the following formula: 4 g/L of copper sulfate, 0.12 g/L of sodium iodide, 5 g/L of manganese sulfate, 0.1 g/L of sodium molybdate, 0.04 g/L of boric acid, 0.6 g/L of cobalt chloride, 18 g/L of zinc chloride, 38 g/L of ferrous sulfate, 0.3 g/L of biotin, and 3 g/L of sulfuric acid.

In the secondary seed culture, when the initial dissolved oxygen was at 100%, the stirring speed was set to 180 rpm, the temperature was 32°C, and the aeration rate of compressed air was 0.2 vvm; when the dissolved oxygen ranged from 80% to 100%, the stirring speed was set to 220 rpm, the temperature was 32°C, and the aeration rate of compressed air was 0.3 vvm; when the dissolved oxygen ranged from 60% to 80%, the stirring speed was set to 280 rpm, the temperature was 32°C, and the aeration rate of compressed air was 0.4 vvm; when the dissolved oxygen ranged from 40% to 60%, the stirring speed was set to 350 rpm, the temperature was 32°C, and the aeration rate of compressed air was 0.5 vvm; when the dissolved oxygen ranged from 20% to 40%, the stirring speed was set to 440 rpm, the temperature was 31°C, and the aeration rate of compressed air was 0.6 vvm; when the dissolved oxygen ranged from 10% to 20%, the stirring speed was set to 520 rpm, the temperature was 31°C, and the aeration rate of compressed air was 0.7 vvm; when the dissolved oxygen ranged from 0% to 10%, the stirring speed was set to 600 rpm, the temperature was 30°C, and the aeration rate of compressed air was 0.8 vvm; when the dissolved oxygen was at 0%, the stirring speed was set to 750 rpm, the temperature was 30°C, and the aeration rate of compressed air was 1 vvm.

The duration for the secondary seed culture was 10 h, with an OD value of about 14, resulting in the secondary seed.

(3) High-density fermentation

40 L of fermentation culture medium was prepared, comprising the following components: 15 g/L of sodium acetate, 2.6% of anhydrous glucose, 1.8% of yeast powder, 2.8% of casein peptone, 1.2% of magnesium sulfate, 1.4% of thiamine hydrochloride, 1% of betaine, 6.8% of ammonium sulfate, 7% of potassium dihydrogen phosphate, 4% of disodium hydrogen phosphate, and 0.2% of acetone;

the fermentation culture medium was added to a 100 L fermentation tank, and sterilized with steam through the fermentation tank, after cooling to 32°C, the secondary seed was added to the 100 L fermentation tank at an inoculum size of 5%;

400 mL of trace elements after sterilization and filtration were then added to the culture medium in the fermentation tank, with the following formula: 8 g/L of copper sulfate, 0.16 g/L of sodium iodide, 6 g/L of manganese sulfate, 0.2 g/L of sodium molybdate, 0.08 g/L of boric acid, 0.8 g/L of cobalt chloride, 21 g/L of zinc chloride, 41 g/L of ferrous sulfate, 0.45 g/L of biotin, and 5.2 g/L of sulfuric acid.

The supplemented carbon source and nitrogen source were prepared. The carbon source was acetic acid, and the nitrogen source was a sterilized 40% urea solution.

In high-density fermentation, the growth environment of the thallus was adjusted to achieve optimal conditions for thallus growth and single-cell protein synthesis by regulating the pH range, temperature, rotation speed, carbon to nitrogen ratio, dissolved oxygen, and gas flow rate during different stages of the process. The specifics were as follows:

under the conditions of $0 \le$ OD value <100, a controlled temperature of 32°C, a pH of 6.5, an aeration rate of compressed air of 0.6 vvm, a rotation speed of 150 rpm, a carbon source feeding rate of 0.8 L/h, and a controlled carbon to nitrogen ratio of 30, the thallus grew rapidly;

under the conditions of $100 \le$ OD value < 300, a controlled temperature of 31°C, a pH of 6.6, an aeration rate of compressed air of 1 vvm, a rotation speed of 200 rpm, a carbon source feeding rate of 1.2 L/h, and a controlled carbon to nitrogen ratio of 30, the thallus grew rapidly;

under the conditions of $300 \le$ OD value < 500, a controlled temperature of 30°C, a pH of 6.7, an aeration rate of a mixed gas (air: pure oxygen at a ratio of 2:1) of 1.2 vvm, a rotation speed of 300 rpm, a carbon source feeding rate of 1.6 L/h, and a controlled carbon to nitrogen ratio of 30, the thallus grew rapidly;

under the conditions of an OD value of 500 to 700, a controlled temperature of 29°C, a pH of 6.8, an aeration rate of a mixed gas (air: pure oxygen at a ratio of 1:1) of 1.6 vvm, a rotation speed of 500 rpm, a carbon source feeding rate of 2.2 L/h, and a controlled carbon to nitrogen ratio of 12, the growth rate of the thallus was normal, while single-cell protein was synthesized simultaneously;

under the conditions of an OD value greater than 700, a controlled temperature of 28°C, a pH of 7, an aeration rate of a mixed gas (air: pure oxygen at a ratio of 1:2) of 2.0 vvm, a rotation speed of 700 rpm, a carbon source feeding rate of 2.5 L/h, and a controlled carbon to nitrogen ratio of 2, the growth rate of the thallus was slow, allowing for sufficient synthesis of single-cell protein.

After 30 h of cultivation, the OD value reached 786, indicating slow growth of the thallus. The fermentation broth was then released from the fermentation tank.

(4) Post-treatment

The fermentation broth was centrifuged and washed with water, then spray dried to obtain single-cell protein with a dry weight of 238 g/L, a yield of 7.9 g/(L·h), and a crude protein content of 60%.

Example 3

A method for producing single-cell protein by means of high-density fermentation, specifically comprising the following steps:

(1) Primary seed culture:

200 mL of primary seed culture medium was prepared, comprising the following components: 8 g/L of sodium acetate, 1.8% of anhydrous glucose, 1.2% of yeast powder, and 2.2% of casein peptone; the primary seed culture medium was sterilized at 121°C for 20 min in a sterilizer. After sterilization, the primary seed culture medium was placed in a super clean bench. After returning to room temperature, the thawed Yarrowia lipolytica strain cryopreservation solution was added to the primary seed culture medium and placed in a shaker, and then cultivated for 18 h at a temperature of 32°C and a rotation speed of 180 rpm, with an OD value of about 5.8, whereby the primary seed was obtained;

(2) Secondary seed culture:

2L of secondary seed culture medium was prepared, comprising the following components: 15g/L of sodium acetate, 2.2% of anhydrous glucose, 1.5% of yeast powder, 2.5% of casein peptone, 1.2% of magnesium sulfate, and 0.6% of thiamine hydrochloride;

the secondary seed culture medium was added to a 5L fermentation tank, and sterilized in a sterilizer at 121°C for 20 min, after cooling to 32°C, the primary seed was added to the secondary seed culture medium fermentation tank at an inoculum size of 7.5% through a flame inoculation;

20 mL of trace element additive after sterilization and filtration was then added, with the following formula: 4 g/L of copper sulfate, 0.12 g/L of sodium iodide, 5 g/L of manganese sulfate, 0.1 g/L of sodium molybdate, 0.04 g/L of boric acid, 0.6 g/L of cobalt chloride, 18 g/L of zinc chloride, 38 g/L of ferrous sulfate, 0.3 g/L of biotin, and 3 g/L of sulfuric acid;

in the secondary seed culture, when the initial dissolved oxygen was at 100%, the stirring speed was set to 180 rpm, the temperature was 32°C, and the aeration rate of compressed air was 0.2 vvm; when the dissolved oxygen ranged from 80% to 100%, the stirring speed was set to 220 rpm, the temperature was 32°C, and the aeration rate of compressed air was 0.3 vvm; when the dissolved oxygen ranged from 60% to 80%, the stirring speed was set to 280 rpm, the temperature was 32°C, and the aeration rate of compressed air was 0.4 vvm; when the dissolved oxygen ranged from 40% to 60%, the stirring speed was set to 350 rpm, the temperature was 32°C, and the aeration rate of compressed air was 0.5 vvm; when the dissolved oxygen ranged from 20% to 40%, the stirring speed was set to 440 rpm, the temperature was 31°C, and the aeration rate of compressed air was 0.6 vvm; when the dissolved oxygen ranged from 10% to 20%, the stirring speed was set to 520 rpm, the temperature was 31°C, and the aeration rate of compressed air was 0.7 vvm; when the dissolved oxygen ranged from 0% to 10%, the stirring speed was set to 600 rpm, the temperature was 30°C, and the aeration rate of compressed air was 0.8 vvm; when the dissolved oxygen was at 0%, the stirring speed was set to 800 rpm, the temperature was 30°C, and the aeration rate of compressed air was 1.2 vvm.

The duration for the secondary seed culture was 12 h, with an OD value of about 16, resulting in the secondary seed.

(3) High-density fermentation

40 L of fermentation culture medium was prepared, comprising the following components: 15 g/L of sodium acetate, 2.6% of anhydrous glucose, 1.8% of yeast powder, 2.8% of casein peptone, 1.2% of magnesium sulfate, 1.4% of thiamine hydrochloride, 1% of betaine, 6.8% of ammonium sulfate, 7% of potassium dihydrogen phosphate, 4% of disodium hydrogen phosphate, and 0.2% of acetone;

the fermentation culture medium was added to a 100 L fermentation tank, and sterilized with steam through the fermentation tank, after cooling to 32°C, the secondary seed was added to the 100 L fermentation tank at an inoculum size of 5%;

400 mL of trace elements after sterilization and filtration were then added to the culture medium in the fermentation tank, with the following formula: 8 g/L of copper sulfate, 0.16 g/L of sodium iodide, 6 g/L of manganese sulfate, 0.2 g/L of sodium molybdate, 0.08 g/L of boric acid, 0.8 g/L of cobalt chloride, 21 g/L of zinc chloride, 41 g/L of ferrous sulfate, 0.45 g/L of biotin, and 5.2 g/L of sulfuric acid.

The supplemented carbon source and nitrogen source were prepared. The carbon source was acetic acid, and the nitrogen source was a sterilized 40% urea solution;

in high-density fermentation, the growth environment of the thallus was adjusted to achieve optimal conditions for thallus growth and single-cell protein synthesis by regulating the pH range, temperature, rotation speed, carbon to nitrogen ratio, dissolved oxygen, and gas flow rate during different stages of the process. The specifics were as follows:

under the conditions of $0 \leq$ OD value <100, a controlled temperature of 32°C, a pH of 6.5, an aeration rate of compressed air of 0.6 vvm, a rotation speed of 150 rpm, a carbon source feeding rate of 0.8 L/h, and a controlled carbon to nitrogen ratio of 50, the thallus grew rapidly;

under the conditions of $100 \leq$ OD value < 300, a controlled temperature of 31°C, a pH of 6.6, an aeration rate of compressed air of 1 vvm, a rotation speed of 200 rpm, a carbon source feeding rate of 1.2 L/h, and a controlled carbon to nitrogen ratio of 50, the thallus grew rapidly;

under the conditions of $300 \leq$ OD value < 500, a controlled temperature of 30°C, a pH of 6.7, an aeration rate of a mixed gas (air: pure oxygen at a ratio of 2:1) of 1.2 vvm, a rotation speed of 300 rpm, a carbon source feeding rate of 1.6 L/h, and a controlled carbon to nitrogen ratio of 50, the thallus grew rapidly;

under the conditions of an OD value of 500 to 700, a controlled temperature of 29°C, a pH of 6.8, an aeration rate of a mixed gas (air: pure oxygen at a ratio of 1:1) of 1.6 vvm, a rotation speed of 500 rpm, a carbon source feeding rate of 2.2 L/h, and a controlled carbon to nitrogen ratio of 18, the growth rate of the thallus was normal, while single-cell protein was synthesized simultaneously;

under the conditions of an OD value greater than 700, a controlled temperature of 28°C, a pH of 7, an aeration rate of a mixed gas (air: pure oxygen at a ratio of 1:2) of 2.0 vvm, a rotation speed of 700 rpm, a carbon source feeding rate of 2.5 L/h, and a controlled carbon to nitrogen ratio of 1, the growth rate of the thallus was slow, allowing for sufficient synthesis of single-cell protein.

After 30 h of cultivation, the OD value reached 792, indicating slow growth of the thallus. The fermentation broth was then released from the fermentation tank.

(4) Post-treatment

The fermentation broth was centrifuged and washed with water, then spray dried to obtain single-cell protein with a dry weight of 243 g/L, a yield of 8.1 g/(L·h), and a crude protein content of 61%.

Example 4

A method for producing single-cell protein by means of high-density fermentation, specifically comprising the following steps:

(1) Primary seed culture: the same as step (1) in Example 1, to obtain the primary seed.
(2) Secondary seed culture: the same as step (2) in Example 1, to obtain the secondary seed.
(3) High-density fermentation

40 L of fermentation culture medium was prepared, comprising the following components: 15 g/L of sodium acetate, 2.6% of anhydrous glucose, 1.8% of yeast powder, 2.8% of casein peptone, 1.2% of magnesium sulfate, 1.4% of thiamine hydrochloride, 1% of betaine, 6.8% of ammonium sulfate, 7% of potassium dihydrogen phosphate, 4% of disodium hydrogen phosphate, and 0.2% of acetone;

the fermentation culture medium was added to a 100 L fermentation tank, and sterilized with steam through the fermentation tank, after cooling to 32°C, the secondary seed was added to the 100 L fermentation tank at an inoculum size of 5%;

400 mL of trace elements after sterilization and filtration were then added to the culture medium in the fermentation tank, with the following formula: 8 g/L of copper sulfate, 0.16 g/L of sodium iodide, 6 g/L of manganese sulfate, 0.2 g/L of sodium molybdate, 0.08 g/L of boric acid, 0.8 g/L of cobalt chloride, 21 g/L of zinc chloride, 41 g/L of ferrous sulfate, 0.45 g/L of biotin, and 5.2 g/L of sulfuric acid.

The supplemented carbon source and nitrogen source were prepared. The carbon source was acetic acid, and the nitrogen source was a sterilized 30% ammonium sulfate solution.

In high-density fermentation, the growth environment of the thallus was adjusted to achieve optimal conditions for thallus growth and single-cell protein synthesis by regulating the pH range, temperature, rotation speed, carbon to nitrogen ratio, dissolved oxygen, and gas flow rate during different stages of the process. The specifics were as follows:

under the conditions of $0 \leq$ OD value <100, a controlled temperature of 32°C, a pH of 6.5, an aeration rate of compressed air of 0.6 vvm, a rotation speed of 150 rpm, a carbon source feeding rate of 0.8 L/h, and a controlled carbon to nitrogen ratio of 20, the thallus grew rapidly;

under the conditions of $100 \leq$ OD value < 300, a controlled temperature of 31°C, a pH of 6.6, an aeration rate of compressed air of 1 vvm, a rotation speed of 200 rpm, a carbon source feeding rate of 1.2 L/h, and a controlled carbon to nitrogen ratio of 20, the thallus grew rapidly;

under the conditions of $300 \leq$ OD value < 500, a controlled temperature of 30°C, a pH of 6.7, an aeration rate of a mixed gas (air: pure oxygen at a ratio of 2:1) of 1.2 vvm, a rotation speed of 300 rpm, a carbon source feeding rate of 1.6 L/h, and a controlled carbon to nitrogen ratio of 20, the thallus grew rapidly;

under the conditions of an OD value of 500 to 700, a controlled temperature of 29°C, a pH of 6.8, an aeration rate of a mixed gas (air: pure oxygen at a ratio of 1:1) of 1.6 vvm, a rotation speed of 500 rpm, a carbon source feeding rate of 2.2 L/h, and a controlled carbon to nitrogen ratio of 10, the growth rate of the thallus was normal, while single-cell protein was synthesized simultaneously;

After 48 h of cultivation, the OD value reached 523, indicating slow growth of the thallus. The fermentation broth was then released from the fermentation tank.

(4) Post-treatment

The fermentation broth was centrifuged and washed with water, then spray dried to obtain single-cell protein with a dry weight of 180 g/L, a yield of 3.75 g/(L·h), and a crude protein content of 41%.

Example 5

A method for producing single-cell protein by means of high-density fermentation, specifically comprising the following steps:

(1) Primary seed culture: the same as step (1) in Example 1, to obtain the primary seed.
(2) Secondary seed culture: the same as step (2) in Example 1, to obtain the secondary seed.
(3) High-density fermentation

40 L of fermentation culture medium was prepared, comprising the following components: 15 g/L of sodium acetate, 2.6% of anhydrous glucose, 1.8% of yeast powder, 2.8% of casein peptone, 1.2% of magnesium sulfate, 1.4% of thiamine hydrochloride, 1% of betaine, 6.8% of ammonium sulfate, 7% of potassium dihydrogen phosphate, 4% of disodium hydrogen phosphate, and 0.2% of acetone;
the fermentation culture medium was added to a 100 L fermentation tank, and sterilized with steam through the fermentation tank, after cooling to 32°C, the secondary seed was added to the 100 L fermentation tank at an inoculum size of 5%;
400 mL of trace elements after sterilization and filtration were then added to the culture medium in the fermentation tank, with the following formula: 8 g/L of copper sulfate, 0.16 g/L of sodium iodide, 6 g/L of manganese sulfate, 0.2 g/L of sodium molybdate, 0.08 g/L of boric acid, 0.8 g/L of cobalt chloride, 21 g/L of zinc chloride, 41 g/L of ferrous sulfate, 0.45 g/L of biotin, and 5.2 g/L of sulfuric acid.

The supplemented carbon source and nitrogen source were prepared. The carbon source was acetic acid, and the nitrogen source was a sterilized 20% ammonium nitrate solution;
in high-density fermentation, the growth environment of the thallus was adjusted to achieve optimal conditions for thallus growth and single-cell protein synthesis by regulating the pH range, temperature, rotation speed, carbon to nitrogen ratio, dissolved oxygen, and gas flow rate during different stages of the process. The specifics were as follows:

under the conditions of $0 \leq$ OD value <100, a controlled temperature of 32°C, a pH of 6.5, an aeration rate of compressed air of 0.6 vvm, a rotation speed of 150 rpm, a carbon source feeding rate of 0.8 L/h, and a controlled carbon to nitrogen ratio of 20, the thallus grew rapidly;
under the conditions of $100 \leq$ OD value < 300, a controlled temperature of 31°C, a pH of 6.6, an aeration rate of compressed air of 1 vvm, a rotation speed of 200 rpm, a carbon source feeding rate of 1.2 L/h, and a controlled carbon to nitrogen ratio of 20, the thallus grew rapidly;
under the conditions of $300 \leq$ OD value < 500, a controlled temperature of 30°C, a pH of 6.7, an aeration rate of a mixed gas (air: pure oxygen at a ratio of 2:1) of 1.2 vvm, a rotation speed of 300 rpm, a carbon source feeding rate of 1.6 L/h, and a controlled carbon to nitrogen ratio of 20, the thallus grew rapidly;

After 52 h of cultivation, the OD value reached about 478, indicating slow growth of the thallus. The fermentation broth was then released from the fermentation tank.

(4) Post-treatment

The fermentation broth was centrifuged and washed with water, then spray dried to obtain single-cell protein with a dry weight of 160 g/L, a yield of 3.08 g/(L·h), and a crude protein content of 38%.

Example 6

A method for producing single-cell protein by means of high-density fermentation, specifically comprising the following steps:

(1) Primary seed culture: the same as step (1) in Example 1, to obtain the primary seed.
(2) Secondary seed culture: the same as step (2) in Example 1, to obtain the secondary seed.
(3) High-density fermentation

40 L of fermentation culture medium was prepared, comprising the following components: 15 g/L of sodium acetate, 2.6% of anhydrous glucose, 1.8% of yeast powder, 2.8% of casein peptone, 1.2% of magnesium sulfate, 1.4% of thiamine hydrochloride, 1% of betaine, 6.8% of ammonium sulfate, 7% of potassium dihydrogen phosphate, 4% of disodium hydrogen phosphate, and 0.2% of acetone;

the fermentation culture medium was added to a 100 L fermentation tank, and sterilized with steam through the fermentation tank, after cooling to 32°C, the secondary seed was added to the 100 L fermentation tank at an inoculum size of 5%;

400 mL of trace elements after sterilization and filtration were then added to the culture medium in the fermentation tank, with the following formula: 8 g/L of copper sulfate, 0.16 g/L of sodium iodide, 6 g/L of manganese sulfate, 0.2 g/L of sodium molybdate, 0.08 g/L of boric acid, 0.8 g/L of cobalt chloride, 21 g/L of zinc chloride, 41 g/L of ferrous sulfate, 0.45 g/L of biotin, and 5.2 g/L of sulfuric acid.

The supplemented carbon source and nitrogen source were prepared. The carbon source was acetic acid, and the nitrogen source was a sterilized 28% ammonia solution;

in high-density fermentation, the growth environment of the thallus was adjusted to achieve optimal conditions for thallus growth and single-cell protein synthesis by regulating the pH range, temperature, rotation speed, carbon to nitrogen ratio, dissolved oxygen, and gas flow rate during different stages of the process. The specifics were as follows:

under the conditions of $0 \leq OD$ value < 100, a controlled temperature of 32°C, a pH of 6.5, an aeration rate of compressed air of 0.6 vvm, a rotation speed of 150 rpm, a carbon source feeding rate of 0.8 L/h, and a controlled carbon to nitrogen ratio of 20, the thallus grew rapidly;

under the conditions of $100 \leq OD$ value < 300, a controlled temperature of 31°C, a pH of 6.6, an aeration rate of compressed air of 1 vvm, a rotation speed of 200 rpm, a carbon source feeding rate of 1.2 L/h, and a controlled carbon to nitrogen ratio of 20, the thallus grew rapidly;

under the conditions of $300 \leq OD$ value < 500, a controlled temperature of 30°C, a pH of 6.7, an aeration rate of a mixed gas (air: pure oxygen at a ratio of 2:1) of 1.2 vvm, a rotation speed of 300 rpm, a carbon source feeding rate of 1.6 L/h, and a controlled carbon to nitrogen ratio of 20, the thallus grew rapidly;

under the conditions of an OD value of 500 to 700, a controlled temperature of 29°C, a pH of 6.8, an aeration rate of a mixed gas (air: pure oxygen at a ratio of 1:1) of 1.6 vvm, a rotation speed of 500 rpm, a carbon source feeding rate of 2.2 L/h, and a controlled carbon to nitrogen ratio of 10, the growth rate of the thallus was normal, while single-cell protein was synthesized simultaneously;

After 50 h of cultivation, the OD value reached about 585, indicating slow growth of the thallus. The fermentation broth was then released from the fermentation tank.

(4) Post-treatment

The fermentation broth was centrifuged and washed with water, then spray dried to obtain single-cell protein with a dry weight of 190 g/L, a yield of 3.8 g/(L·h), and a crude protein content of 42%.

A comparison of Example 1 and Examples 4 to 6 demonstrates that in the high-density fermentation process of Example 1, the supplementation of urea as a nitrogen source and acetic acid as a carbon source can more effectively promote the rapid growth of the thallus and the synthesis of proteins. Wherein, urea, as a nitrogen source, is more easily absorbed by the thallus, which is conducive to protein synthesis. The supplemented nitrogen sources in Examples 4 to 6 are not conducive to thallus absorption, resulting in slow growth, which not only prolongs the fermentation period but also reduces the yield of single-cell protein obtained.

Example 7

A method for producing single-cell protein by means of high-density fermentation, specifically comprising the following steps:

(1) Primary seed culture: the same as step (1) in Example 1, to obtain the primary seed.
(2) Secondary seed culture: the same as step (2) in Example 1, to obtain the secondary seed.
(3) High-density fermentation

40 L of fermentation culture medium was prepared, comprising the following components: 15 g/L of sodium acetate, 2.6% of anhydrous glucose, 1.8% of yeast powder, 2.8% of casein peptone, 1.2% of magnesium sulfate, 1.4% of thiamine hydrochloride, 1% of betaine, 6.8% of ammonium sulfate, 7% of potassium dihydrogen phosphate, 4% of disodium hydrogen phosphate, and 0.2% of acetone;

the fermentation culture medium was added to a 100 L fermentation tank, and sterilized with steam through the fermentation tank, after cooling to 32°C, the secondary seed was added to the 100 L fermentation tank at an inoculum size of 5%;

400 mL of trace elements after sterilization and filtration were then added to the culture medium in the fermentation tank, with the following formula: 8 g/L of copper sulfate, 0.16 g/L of sodium iodide, 6 g/L of manganese sulfate, 0.2 g/L of sodium molybdate, 0.08 g/L of boric acid, 0.8 g/L of cobalt chloride, 21 g/L of zinc chloride, 41 g/L of ferrous sulfate, 0.45 g/L of biotin, and 5.2 g/L of sulfuric acid.

The supplemented carbon source and nitrogen source were prepared. The carbon source was acetic acid, and the nitrogen source was a sterilized 40% urea solution.

In high-density fermentation, the growth environment of the thallus was adjusted to achieve optimal conditions for thallus growth and single-cell protein synthesis by regulating the pH range, temperature, rotation speed, carbon to nitrogen ratio, dissolved oxygen, and gas flow rate during different stages of the process. The specifics were as follows:

under the conditions of $0 \le$ OD value <100, a controlled temperature of 32°C, a pH of 6.5, an aeration rate of compressed air of 0.6 vvm, a rotation speed of 150 rpm, a carbon source feeding rate of 0.8 L/h, and a maintained constant carbon to nitrogen ratio of 20, the thallus grew rapidly;

under the conditions of $100 \le$ OD value < 300, a controlled temperature of 31°C, a pH of 6.6, an aeration rate of compressed air of 1 vvm, a rotation speed of 200 rpm, a carbon source feeding rate of 1.2 L/h, and a maintained constant carbon to nitrogen ratio of 20, the thallus grew rapidly;

under the conditions of $300 \le$ OD value < 500, a controlled temperature of 30°C, a pH of 6.7, an aeration rate of a mixed gas (air: pure oxygen at a ratio of 2:1) of 1.2 vvm, a rotation speed of 300 rpm, a carbon source feeding rate of 1.6 L/h, and a maintained constant carbon to nitrogen ratio of 20, the thallus grew rapidly;

under the conditions of an OD value of 500 to 700, a controlled temperature of 29°C, a pH of 6.8, an aeration rate of a mixed gas (air: pure oxygen at a ratio of 1:1) of 1.6 vvm, a rotation speed of 500 rpm, a carbon source feeding rate of 2.2 L/h, and a maintained constant carbon to nitrogen ratio of 20, the growth rate of the thallus was normal, while single-cell protein was synthesized simultaneously;

After 36 h of cultivation, the OD value reached about 641, indicating slow growth of the thallus. The fermentation broth was then released from the fermentation tank.

(4) Post-treatment

The fermentation broth was centrifuged and washed with water, then spray dried to obtain single-cell protein with a dry weight of 210 g/L, a yield of 5.8 g/(L·h), and a crude protein content of 35%.

A comparison of Example 1 and Example 7 demonstrates that in the high-density fermentation process of Example 1, the carbon to nitrogen ratio was adjusted according to the fermentation stage. When the carbon to nitrogen ratio was greater than 18, the growth of the thallus was vigorous, and when the carbon to nitrogen ratio was less than 5, the growth of the thallus slowed down, while the speed of protein synthesis increased. By adjusting the carbon to nitrogen ratio, the fastest growth of thallus and the highest content of crude protein were achieved. In Example 7, the high-density fermentation process maintained a constant high carbon to nitrogen ratio, resulting in a slower speed of protein synthesis, and ultimately a lower content of crude protein was obtained.

Example 8

A method for producing single-cell protein by means of high-density fermentation, specifically comprising the following steps:

(1) Primary seed culture: the same as step (1) in Example 1, to obtain the primary seed.
(2) Secondary seed culture: the same as step (2) in Example 1, to obtain the secondary seed.
(3) High-density fermentation

40 L of fermentation culture medium was prepared, comprising the following components: 15 g/L of sodium acetate, 2.6% of anhydrous glucose, 1.8% of yeast powder, 2.8% of casein peptone, 1.2% of magnesium sulfate, 1.4% of thiamine hydrochloride, 1% of betaine, 6.8% of ammonium sulfate, 7% of potassium dihydrogen phosphate, 4% of disodium hydrogen phosphate, and 0.2% of acetone;

the fermentation culture medium was added to a 100 L fermentation tank, and sterilized with steam through the fermentation tank, after cooling to 32°C, the secondary seed was added to the 100 L fermentation tank at an inoculum size of 5%;

400 mL of trace elements after sterilization and filtration were then added to the culture medium in the fermentation tank, with the following formula: 8 g/L of copper sulfate, 0.16 g/L of sodium iodide, 6 g/L of manganese sulfate, 0.2 g/L of sodium molybdate, 0.08 g/L of boric acid, 0.8 g/L of cobalt chloride, 21 g/L of zinc chloride, 41 g/L of ferrous sulfate, 0.45 g/L of biotin, and 5.2 g/L of sulfuric acid.

The supplemented carbon source and nitrogen source were prepared. The carbon source was acetic acid, and the nitrogen source was a sterilized 40% urea solution.

In high-density fermentation, the growth environment of the thallus was adjusted to achieve optimal conditions for thallus growth and single-cell protein synthesis by regulating the pH range, temperature, rotation speed, carbon to nitrogen ratio, dissolved oxygen, and gas flow rate during different stages of the process. The specifics were as follows:

under the conditions of $0 \leq OD$ value <100, a controlled temperature of 32°C, a pH of 6.5, an aeration rate of compressed air of 0.6 vvm, a rotation speed of 150 rpm, a carbon source feeding rate of 0.8 L/h, and a maintained constant carbon to nitrogen ratio of 12, the thallus grew rapidly;

under the conditions of $100 \leq OD$ value < 300, a controlled temperature of 31°C, a pH of 6.6, an aeration rate of compressed air of 1 vvm, a rotation speed of 200 rpm, a carbon source feeding rate of 1.2 L/h, and a maintained constant carbon to nitrogen ratio of 12, the thallus grew rapidly;

under the conditions of $300 \leq OD$ value < 500, a controlled temperature of 30°C, a pH of 6.7, an aeration rate of a mixed gas (air: pure oxygen at a ratio of 2:1) of 1.2 vvm, a rotation speed of 300 rpm, a carbon source feeding rate of 1.6 L/h, and a maintained constant carbon to nitrogen ratio of 12, the thallus grew rapidly;

under the conditions of an OD value of 500 to 700, a controlled temperature of 29°C, a pH of 6.8, an aeration rate of a mixed gas (air: pure oxygen at a ratio of 1:1) of 1.6 vvm, a rotation speed of 500 rpm, a carbon source feeding rate of 2.2 L/h, and a maintained constant carbon to nitrogen ratio of 12, the growth rate of the thallus was normal, while single-cell protein was synthesized simultaneously.

After 46 h of cultivation, the OD value reached about 536, indicating slow growth of the thallus. The fermentation broth was then released from the fermentation tank.

(4) Post-treatment

The fermentation broth was centrifuged and washed with water, then spray dried to obtain single-cell protein with a dry weight of 186 g/L, a yield of 4.04 g/(L·h), and a crude protein content of 55%.

A comparison of Example 1 and Example 8 demonstrates that in the high-density fermentation process of Example 1, the carbon to nitrogen ratio was regulated according to the fermentation stage. When the carbon to nitrogen ratio was greater than 18, the growth of the thallus was vigorous, and when the carbon to nitrogen ratio was less than 5, the growth of the thallus slowed down, while the speed of protein synthesis increased. By adjusting the carbon to nitrogen ratio, the fastest growth of thallus and the highest content of crude protein were achieved. In Example 8, the high-density fermentation process maintained a constant carbon to nitrogen ratio of 12, resulting in slower growth rate of thallus and synthesis rate of protein, as well as reduced yield and efficiency.

Example 9

A method for producing single-cell protein by means of high-density fermentation, specifically comprising the following steps:

(1) Primary seed culture: the same as step (1) in Example 1, to obtain the primary seed.
(2) Secondary seed culture: the same as step (2) in Example 1, to obtain the secondary seed.
(3) High-density fermentation

40 L of fermentation culture medium was prepared, comprising the following components: 15 g/L of sodium acetate, 2.6% of anhydrous glucose, 1.8% of yeast powder, 2.8% of casein peptone, 1.2% of magnesium sulfate, 1.4% of thiamine hydrochloride, 1% of betaine, 6.8% of ammonium sulfate, 7% of potassium dihydrogen phosphate, 4% of disodium hydrogen phosphate, and 0.2% of acetone;

the fermentation culture medium was added to a 100 L fermentation tank, and sterilized with steam through the fermentation tank, after cooling to 32°C, the secondary seed was added to the 100 L fermentation tank at an inoculum size of 5%;

400 mL of trace elements after sterilization and filtration were then added to the culture medium in the fermentation tank, with the following formula: 8 g/L of copper sulfate, 0.16 g/L of sodium iodide, 6 g/L of manganese sulfate, 0.2 g/L of sodium molybdate, 0.08 g/L of boric acid, 0.8 g/L of cobalt chloride, 21 g/L of zinc chloride, 41 g/L of ferrous sulfate, 0.45 g/L of biotin, and 5.2 g/L of sulfuric acid.

The supplemented carbon source and nitrogen source were prepared. The carbon source was acetic acid, and the nitrogen source was a sterilized 40% urea solution.

In high-density fermentation, the growth environment of the thallus was adjusted to achieve optimal conditions for thallus growth and single-cell protein synthesis by regulating the pH range, temperature, rotation speed, carbon to nitrogen ratio, dissolved oxygen, and gas flow rate during different stages of the process. The specifics were as follows:

under the conditions of $0 \leq$ OD value <100, a controlled temperature of 32°C, a pH of 6.5, an aeration rate of compressed air of 0.6 vvm, a rotation speed of 150 rpm, a carbon source feeding rate of 0.8 L/h, and a maintained constant carbon to nitrogen ratio of 2, the thallus grew rapidly;

under the conditions of $100 \leq$ OD value < 300, a controlled temperature of 31°C, a pH of 6.6, an aeration rate of compressed air of 1 vvm, a rotation speed of 200 rpm, a carbon source feeding rate of 1.2 L/h, and a maintained constant carbon to nitrogen ratio of 2, the thallus grew rapidly;

under the conditions of $300 \leq$ OD value < 500, a controlled temperature of 30°C, a pH of 6.7, an aeration rate of a mixed gas (air: pure oxygen at a ratio of 2:1) of 1.2 vvm, a rotation speed of 300 rpm, a carbon source feeding rate of 1.6 L/h, and a maintained constant carbon to nitrogen ratio of 2, the thallus grew rapidly;

under the conditions of an OD value of 500 to 700, a controlled temperature of 29°C, a pH of 6.8, an aeration rate of a mixed gas (air: pure oxygen at a ratio of 1:1) of 1.6 vvm, a rotation speed of 500 rpm, a carbon source feeding rate of 2.2 L/h, and a maintained constant carbon to nitrogen ratio of 2, the growth rate of the thallus was normal, while single-cell protein was synthesized simultaneously;

After 64 h of cultivation, the OD value reached about 528, indicating slow growth of the thallus. The fermentation broth was then released from the fermentation tank.

(4) Post-treatment

The fermentation broth was centrifuged and washed with water, then spray dried to obtain single-cell protein with a dry weight of 172 g/L, a yield of 2.68 g/(L·h), and a crude protein content of 58%.

A comparison of Example 1 and Example 9 demonstrates that in the high-density fermentation process of Example 1, the carbon to nitrogen ratio was regulated according to the fermentation stage. When the carbon to nitrogen ratio was greater than 18, the growth of the thallus was vigorous, and when the carbon to nitrogen ratio was less than 5, the growth of the thallus slowed down, while the speed of protein synthesis increased. By adjusting the carbon to nitrogen ratio, the fastest growth of thallus and the highest content of crude protein were achieved. In Example 9, the high-density fermentation process maintained a constant low carbon to nitrogen ratio, resulting in a slow growth rate of thallus and a long fermentation period.

Comparative Example 1

A method for producing single-cell protein by means of high-density fermentation, specifically comprising the following steps:

(1) Primary seed culture:
200 mL of primary seed culture medium was prepared, comprising the following components: 1.8% of anhydrous glucose, 1.2% of yeast powder, and 2.2% of casein peptone; the primary seed culture medium was sterilized at 121°C for 20 min in a sterilizer. After sterilization, the primary seed culture medium was placed in a super clean bench. After returning to room temperature, the thawed Yarrowia lipolytica strain cryopreservation solution was added to the primary seed culture medium and placed in a shaker, and then cultivated for 16 h at a temperature of 32°C and a rotation speed of 180 rpm, with an OD value of about 4.2, whereby the primary seed was obtained;
(2) Secondary seed culture:

2L of secondary seed culture medium was prepared, comprising the following components: 2.2% of anhydrous glucose, 1.5% of yeast powder, 2.5% of casein peptone, 1.2% of magnesium sulfate, and 0.6% of thiamine hydrochloride;
the secondary seed culture medium was added to a 5L fermentation tank, and sterilized in a sterilizer at 121°C for 20 min, after cooling to 32°C, the primary seed was added to the secondary seed culture medium fermentation tank at an inoculum size of 7.5% through flame inoculation;

20 mL of trace element additive after sterilization and filtration was then added, with the following formula: 4 g/L of copper sulfate, 0.12 g/L of sodium iodide, 5 g/L of manganese sulfate, 0.1 g/L of sodium molybdate, 0.04 g/L of boric acid, 0.6 g/L of cobalt chloride, 18 g/L of zinc chloride, 38 g/L of ferrous sulfate, 0.3 g/L of biotin, and 3 g/L of sulfuric acid.

In the secondary seed culture, when the initial dissolved oxygen was at 100%, the stirring speed was set to 180 rpm, the temperature was 32°C, and the aeration rate of compressed air was 0.2 vvm; when the dissolved oxygen ranged from 80% to 100%, the stirring speed was set to 220 rpm, the temperature was 32°C, and the aeration rate of compressed air was 0.3 vvm; when the dissolved oxygen ranged from 60% to 80%, the stirring speed was set to 280 rpm, the temperature was 32°C, and the aeration rate of compressed air was 0.4 vvm; when the dissolved oxygen ranged from 40% to 60%, the stirring speed was set to 350 rpm, the temperature was 32°C, and the aeration rate of compressed air was 0.5 vvm; when the dissolved oxygen ranged from 20% to 40%, the stirring speed was set to 440 rpm, the temperature was 31°C, and the aeration rate of compressed air was 0.6 vvm; when the dissolved oxygen ranged from 10% to 20%, the stirring speed was set to 520 rpm, the temperature was 31°C, and the aeration rate of compressed air was 0.7 vvm; when the dissolved oxygen ranged from 0% to 10%, the stirring speed was set to 600 rpm, the temperature was 30°C, and the aeration rate of compressed air was 0.8 vvm; when the dissolved oxygen was at 0%, the stirring speed was set to 700 rpm, the temperature was 30°C, and the aeration rate of compressed air was 0.9 vvm.

The duration for the secondary seed culture was 8 h, with an OD value of about 12, resulting in the secondary seed.

(3) High-density fermentation

40 L of fermentation culture medium was prepared, comprising the following components: 2.6% of anhydrous glucose, 1.8% of yeast powder, 2.8% of casein peptone, 1.2% of magnesium sulfate, 1.4% of thiamine hydrochloride, 1% of betaine, 6.8% of ammonium sulfate, 7% of potassium dihydrogen phosphate, 4% of disodium hydrogen phosphate, and 0.2% of acetone;

the fermentation culture medium was added to a 100 L fermentation tank, and sterilized with steam through the fermentation tank, after cooling to 32°C, the secondary seed was added to the 100 L fermentation tank at an inoculum size of 5%;

400 mL of trace elements after sterilization and filtration were then added to the culture medium in the fermentation tank, with the following formula: 8 g/L of copper sulfate, 0.16 g/L of sodium iodide, 6 g/L of manganese sulfate, 0.2 g/L of sodium molybdate, 0.08 g/L of boric acid, 0.8 g/L of cobalt chloride, 21 g/L of zinc chloride, 41 g/L of ferrous sulfate, 0.45 g/L of biotin, and 5.2 g/L of sulfuric acid.

The supplemented carbon source and nitrogen source were prepared. The carbon source was acetic acid, and the nitrogen source was a sterilized 40% urea solution.

In high-density fermentation, the growth environment of the thallus was adjusted to achieve optimal conditions for thallus growth and single-cell protein synthesis by regulating the pH range, temperature, rotation speed, carbon to nitrogen ratio, dissolved oxygen, and gas flow rate during different stages of the process. The specifics were as follows:

under the conditions of $0 \leq$ OD value <100, a controlled temperature of 32°C, a pH of 6.5, an aeration rate of compressed air of 0.6 vvm, a rotation speed of 150 rpm, a carbon source feeding rate of 0.8 L/h, and a controlled carbon to nitrogen ratio of 20, the thallus grew rapidly;

under the conditions of $100 \leq$ OD value < 300, a controlled temperature of 31°C, a pH of 6.6, an aeration rate of compressed air of 1 vvm, a rotation speed of 200 rpm, a carbon source feeding rate of 1.2 L/h, and a controlled carbon to nitrogen ratio of 20, the thallus grew rapidly;

under the conditions of $300 \leq$ OD value < 500, a controlled temperature of 30°C, a pH of 6.7, an aeration rate of a mixed gas (air: pure oxygen at a ratio of 2:1) of 1.2 vvm, a rotation speed of 300 rpm, a carbon source feeding rate of 1.6 L/h, and a controlled carbon to nitrogen ratio of 20;

After 68 h of cultivation, the OD value reached about 372, indicating slow growth of the thallus. The fermentation broth was then released from the fermentation tank.

(4) Post-treatment

The fermentation broth was centrifuged and washed with water, then spray dried to obtain single-cell protein with a dry weight of 120 g/L, a yield of 1.7 g/(L·h), and a crude protein content of 60%.

As sodium acetate was not included in the carbon sources of the primary seed culture medium, the secondary seed culture medium, and the fermentation culture medium of Comparative Example 1, the thallus grew slowly when exposed to acetic acid during the high-density fermentation stage. Additional time was required for adaptation to the acetate environment to establish an acetic acid metabolic pathway, resulting in longer growth duration, difficulty in achieving high density, and low yield and efficiency.

Comparative Example 2

A method for producing single-cell protein by means of high-density fermentation, specifically comprising the following steps:

(1) Primary seed culture: the same as step (1) in Example 1, to obtain the primary seed.
(2) Secondary seed culture: the same as step (2) in Example 1, to obtain the secondary seed.
(3) High-density fermentation

40 L of fermentation culture medium was prepared, comprising the following components: 15 g/L of sodium acetate, 2.6% of anhydrous glucose, 1.8% of yeast powder, 2.8% of casein peptone, 1.2% of magnesium sulfate, 1.4% of thiamine hydrochloride, 1% of betaine, 6.8% of ammonium sulfate, 7% of potassium dihydrogen phosphate, 4% of disodium hydrogen phosphate, and 0.2% of acetone;
the fermentation culture medium was added to a 100 L fermentation tank, and sterilized with steam through the fermentation tank, after cooling to 32°C, the secondary seed was added to the 100 L fermentation tank at an inoculum size of 5%;
400 mL of trace elements after sterilization and filtration were then added to the culture medium in the fermentation tank, with the following formula: 8 g/L of copper sulfate, 0.16 g/L of sodium iodide, 6 g/L of manganese sulfate, 0.2 g/L of sodium molybdate, 0.08 g/L of boric acid, 0.8 g/L of cobalt chloride, 21 g/L of zinc chloride, 41 g/L of ferrous sulfate, 0.45 g/L of biotin, and 5.2 g/L of sulfuric acid.

The supplemented carbon source and nitrogen source were prepared. The carbon source was a sterilized 40% aqueous sodium acetate solution, and the nitrogen source was a sterilized 40% urea solution;

In high-density fermentation, the growth environment of the thallus was adjusted to achieve optimal conditions for thallus growth and single-cell protein synthesis by regulating the pH range, temperature, rotation speed, carbon to nitrogen ratio, dissolved oxygen, and gas flow rate during different stages of the process. The specifics were as follows:

under the conditions of $0 \leq$ OD value <100, a controlled temperature of 32°C, a pH of 6.5, an aeration rate of compressed air of 0.6 vvm, a rotation speed of 150 rpm, a carbon source feeding rate of 0.8 L/h, and a controlled carbon to nitrogen ratio of 20, the thallus grew rapidly;
under the conditions of $100 \leq$ OD value < 300, a controlled temperature of 31°C, a pH of 6.6, an aeration rate of compressed air of 1 vvm, a rotation speed of 200 rpm, a carbon source feeding rate of 1.2 L/h, and a controlled carbon to nitrogen ratio of 20;
After 42 h of cultivation, the OD value reached about 172. The fermentation broth was saponified, and the thallus were lysed and inactivated. The fermentation broth was then subjected to centrifugation, water washing, and spray drying. The resulting single-cell protein exhibited a dry weight of 60 g/L, a yield of 1.4 g/(L·h), and a crude protein content of 38%.

Due to the generation of alkaline substances during the fermentation process, the pH in the fermentation tank gradually increased. In Comparative Example 2, sodium acetate (alkaline) was supplemented as a carbon source during the high-density fermentation process, resulting in a more alkaline pH condition and accumulation of sodium ion, which led to saponification reactions and inhibited the growth of the thallus.

Comparative Example 3

A method for producing single-cell protein by means of high-density fermentation, specifically comprising the following steps:

(1) Primary seed culture:

200 mL of primary seed culture medium was prepared, comprising the following components: 8 g/L of sodium acetate, 1.8% of anhydrous glucose, 1.2% of yeast powder, and 2.2% of casein peptone; the primary seed culture medium was sterilized at 121°C for 20 min in a sterilizer. After sterilization, the primary seed culture medium was placed in a super clean bench. After returning to room temperature, the thawed Schizochytrium strain cryopreservation solution (accession number ATCC 20888, purchased from the American Type Culture Collection) was added to the primary seed culture medium and placed in a shaker, and cultivated for 16 h at a temperature of 32°C and a rotation speed of 180 rpm, with an OD value of about 4.2, whereby the primary seed was obtained;

(2) Secondary seed culture:

2L of secondary seed culture medium was prepared, comprising the following components: 15 g/L of sodium acetate, 2.2% of anhydrous glucose, 1.5% of yeast powder, 2.5% of casein peptone, 1.2% of magnesium sulfate, and 0.6% of thiamine hydrochloride;

the secondary seed culture medium was added to a 5L fermentation tank and sterilized in a sterilizer at 121°C for 20 min, after cooling to 32°C, the primary seed was added to the secondary seed culture medium fermentation tank at an inoculum size of 7.5% through flame inoculation;

20 mL of trace element additive after sterilization and filtration was then added, with the following formula: 4 g/L of copper sulfate, 0.12 g/L of sodium iodide, 5 g/L of manganese sulfate, 0.1 g/L of sodium molybdate, 0.04 g/L of boric acid, 0.6 g/L of cobalt chloride, 18 g/L of zinc chloride, 38 g/L of ferrous sulfate, 0.3 g/L of biotin, and 3 g/L of sulfuric acid;

In the secondary seed culture, when the initial dissolved oxygen was at 100%, the stirring speed was set to 180 rpm, the temperature was 32°C, and the aeration rate of compressed air was 0.2 vvm; when the dissolved oxygen ranged from 80% to 100%, the stirring speed was set to 220 rpm, the temperature was 32°C, and the aeration rate of compressed air was 0.3 vvm; when the dissolved oxygen ranged from 60% to 80%, the stirring speed was set to 280 rpm, the temperature was 32°C, and the aeration rate of compressed air was 0.4 vvm; when the dissolved oxygen ranged from 40% to 60%, the stirring speed was set to 350 rpm, the temperature was 32°C, and the aeration rate of compressed air was 0.5 vvm; when the dissolved oxygen ranged from 20% to 40%, the stirring speed was set to 440 rpm, the temperature was 31°C, and the aeration rate of compressed air was 0.6 vvm; when the dissolved oxygen ranged from 10% to 20%, the stirring speed was set to 520 rpm, the temperature was 31°C, and the aeration rate of compressed air was 0.7 vvm; when the dissolved oxygen ranged from 0% to 10%, the stirring speed was set to 600 rpm, the temperature was 30°C, and the aeration rate of compressed air was 0.8 vvm; when the dissolved oxygen was at 0%, the stirring speed was set to 700 rpm, the temperature was 30°C, and the aeration rate of compressed air was 0.9 vvm.

The duration for the secondary seed culture was 8 h, with an OD value of about 12, resulting in the secondary seed.

(3) High-density fermentation

40 L of fermentation culture medium was prepared, comprising the following components: 15 g/L of sodium acetate, 2.6% of anhydrous glucose, 1.8% of yeast powder, 2.8% of casein peptone, 1.2% of magnesium sulfate, 1.4% of thiamine hydrochloride, 1% of betaine, 6.8% of ammonium sulfate, 7% of potassium dihydrogen phosphate, 4% of disodium hydrogen phosphate, and 0.2% of acetone;

the fermentation culture medium was added to a 100 L fermentation tank and sterilized with steam through the fermentation tank, after cooling to 32°C, the secondary seed was added to the 100 L fermentation tank at an inoculum size of 5%;

400 mL of trace elements after sterilization and filtration were then added to the culture medium in the fermentation tank, with the following formula: 8 g/L of copper sulfate, 0.16 g/L of sodium iodide, 6 g/L of manganese sulfate, 0.2 g/L of sodium molybdate, 0.08 g/L of boric acid, 0.8 g/L of cobalt chloride, 21 g/L of zinc chloride, 41 g/L of ferrous sulfate, 0.45 g/L of biotin, and 5.2 g/L of sulfuric acid.

The supplemented carbon source and nitrogen source were prepared. The carbon source was acetic acid, and the nitrogen source was a sterilized 40% urea solution.

In high-density fermentation, the growth environment of the thallus was adjusted to achieve optimal conditions for thallus growth and single-cell protein synthesis by regulating the pH range, temperature, rotation speed, carbon to nitrogen ratio, dissolved oxygen, and gas flow rate during different stages of the process. The specifics were as follows:

under the conditions of 0 ≤ OD value <100, a controlled temperature of 32°C, a pH of 6.5, an aeration rate of compressed air of 0.6 vvm, a rotation speed of 150 rpm, a carbon source feeding rate of 0.8 L/h, and a controlled carbon to nitrogen ratio of 20, the thallus grew rapidly;

under the conditions of 100 ≤ OD value < 300, a controlled temperature of 31°C, a pH of 6.6, an aeration rate of compressed air of 1 vvm, a rotation speed of 200 rpm, a carbon source feeding rate of 1.2 L/h, and a controlled carbon to nitrogen ratio of 20;

After 96 h of cultivation, the OD value reached about 224, indicating slow growth of the thallus. The fermentation broth was then released from the fermentation tank.

(4) Post-treatment

The fermentation broth was centrifuged and washed with water, then spray dried to obtain single-cell protein with a dry weight of 86 g/L, a yield of 0.9 g/(L·h), and a crude protein content of 28%.

A comparison of Example 1 and Comparative Example 3 demonstrates that the present application uses Yarrowia lipolytica as the strain, which is a non-genetically engineered strain, and is safer than conventional thallus. Yarrowia lipolytica is a microorganism generally recognized as safe (GRAS) by the FDA and is widely used in the food industry. The present application utilizes a specific strain and adopts a specific fermentation method to quickly obtain high-yield single-cell protein. In Comparative Example 3, Schizochytrium is used, which not only results in a long fermentation period but also yields low single-cell protein production.

Comparative Example 4

A method for producing single-cell protein by means of high-density fermentation, specifically comprising the following steps:

(1) Primary seed culture: the same as step (1) in Example 1, to obtain the primary seed.
(2) Secondary seed culture: the same as step (2) in Example 1, to obtain the secondary seed.
(3) High-density fermentation

40 L of fermentation culture medium was prepared, comprising the following components: 15 g/L of sodium acetate, 2.6% of anhydrous glucose, 1.8% of yeast powder, 2.8% of casein peptone, 1.2% of magnesium sulfate, 1.4% of thiamine hydrochloride, 1% of betaine, 6.8% of ammonium sulfate, 7% of potassium dihydrogen phosphate, and 4% of disodium hydrogen phosphate;

the fermentation culture medium was added to a 100 L fermentation tank and sterilized with steam through the fermentation tank, after cooling to 32°C, the secondary seed was added to the 100 L fermentation tank at an inoculum size of 5%;

400 mL of trace elements after sterilization and filtration were then added to the culture medium in the fermentation tank, with the following formula: 8 g/L of copper sulfate, 0.16 g/L of sodium iodide, 6 g/L of manganese sulfate, 0.2 g/L of sodium molybdate, 0.08 g/L of boric acid, 0.8 g/L of cobalt chloride, 21 g/L of zinc chloride, 41 g/L of ferrous sulfate, 0.45 g/L of biotin, and 5.2 g/L of sulfuric acid.

The supplemented carbon source and nitrogen source were prepared. The carbon source was acetic acid, and the nitrogen source was a sterilized 40% urea solution.

In high-density fermentation, the growth environment of the thallus was adjusted to achieve optimal conditions for thallus growth and single-cell protein synthesis by regulating the pH range, temperature, rotation speed, carbon to nitrogen ratio, dissolved oxygen, and gas flow rate during different stages of the process. The specifics were as follows:

under the conditions of 0 ≤ OD value <100, a controlled temperature of 32°C, a pH of 6.5, an aeration rate of compressed air of 0.6 vvm, a rotation speed of 150 rpm, a carbon source feeding rate of 0.8 L/h, and a controlled carbon to nitrogen ratio of 20, the thallus grew rapidly;

under the conditions of 100 ≤ OD value < 300, a controlled temperature of 3 1°C, a pH of 6.6, an aeration rate of compressed air of 1 vvm, a rotation speed of 200 rpm, a carbon source feeding rate of 1.2 L/h, and a controlled carbon to nitrogen ratio of 20, the thallus grew rapidly;

under the conditions of 300 ≤ OD value < 500, a controlled temperature of 30°C, a pH of 6.7, an aeration rate of a mixed gas (air: pure oxygen at a ratio of 2:1) of 1.2 vvm, a rotation speed of 300 rpm, a carbon source feeding rate of 1.6 L/h, and a controlled carbon to nitrogen ratio of 20, the thallus grew rapidly;

under the conditions of an OD value of 500 to 700, a controlled temperature of 29°C, a pH of 6.8, an aeration rate of a mixed gas (air: pure oxygen at a ratio of 1:1) of 1.6 vvm, a rotation speed of 500 rpm, a carbon source feeding rate of 2.2 L/h, and a controlled carbon to nitrogen ratio of 10, the growth rate of the thallus was normal, while single-cell protein was synthesized simultaneously;

After 51 h of cultivation, the OD value reached about 647, indicating slow growth of the thallus. The fermentation broth was then released from the fermentation tank.

(4) Post-treatment

The fermentation broth was centrifuged and washed with water, then spray dried to obtain single-cell protein with a dry weight of 216 g/L, a yield of 4.23 g/(L·h), and a crude protein content of 60%.

A comparison of Example 1 and Comparative Example 4 demonstrates that the fermentation culture medium of the present application contains ketone compounds (preferably acetone), which has catalytic functions on the intermediate products in the acetic acid metabolism pathway, thereby promoting rapid growth of the thallus and shortening the fermentation period. The fermentation culture medium of Comparative Example 4 does not contain ketone compounds, resulting in slow growth of the thallus and prolonged fermentation period.

The applicant declares that the present application illustrates the method for producing single-cell protein by means of high-density fermentation through the aforementioned Examples, but the present application is not limited to the aforementioned process steps, nor does it imply that the present application must rely on the aforementioned process steps for implementation. Those skilled in the art should understand that any improvements to the present application, equivalent substitutions of the raw materials used in the present application, addition of auxiliary components, and selection of specific methods are all within the scope of protection and disclosure of the present application.

**Claims**

1. A method for producing single-cell protein by means of high-density fermentation, including:

   inoculating a Yarrowia lipolytica strain into a seed culture medium for seed culture to obtain a seed liquid;
   inoculating the seed liquid into a fermentation culture medium for high-density fermentation, and supplementing same with acetic acid and a nitrogen source during the process of the high-density fermentation to obtain the single-cell protein;
   wherein a carbon source of the seed culture medium comprises a first acetate, and a carbon source of the fermentation culture medium comprises a second acetate and/or a ketone compound.

2. The method according to claim 1, wherein the first acetate includes any one or a combination of at least two of sodium acetate, potassium acetate and ammonium acetate, preferably sodium acetate.

3. The method according to claim 2, wherein a mass concentration of the first acetate in the seed culture medium is in a range from 5 g/L to 15 g/L.

4. The method according to claim 2 or 3, wherein the seed culture includes a primary seed culture and a secondary seed culture carried out sequentially.

5. The method according to claim 4, wherein a primary seed culture medium used for the primary seed culture includes the following components: 5-15 g/L of first acetate, 1%-3% of glucose, 0.6%-2% of yeast powder and 1.5%-3.2% of casein peptone;

   preferably, the temperature for the primary seed culture ranges from 30°C to 33°C, and the duration ranges from 16 h to 18 h;
   preferably, the primary seed culture is carried out under stirring conditions, with the stirring speed ranging from 100 rpm to 300 rpm;
   preferably, the primary seed culture is processed to an OD value of 4.2 to 6.8, obtaining a primary seed.

6. The method according to claim 4 or 5, wherein a secondary seed culture medium used for the secondary seed culture includes the following components: 5-20 g/L of first acetate, 1%-3% of glucose, 0.6%-2% of yeast powder, 1.5%-3.2% of casein peptone, 0.8%-2.4% of magnesium sulfate and 0.2%-0.8% of thiamine hydrochloride;

   preferably, the secondary seed culture medium further includes a first trace element additive, and the usage amount of the first trace element additive is in a range from 0.8% to 2.6%;

preferably, the first trace element additive includes the following components: 4-8 g/L of copper sulfate, 0.12-0.16 g/L of sodium iodide, 5-6 g/L of manganese sulfate, 0.1-0.2 g/L of sodium molybdate, 0.04-0.08 g/L of boric acid, 0.6-0.8 g/L of cobalt chloride, 18-21 g/L of zinc chloride, 38-41 g/L of ferrous sulfate, 0.3-0.5 g/L of biotin and 3-5.2 g/L of sulfuric acid.

7. The method according to any one of claims 4 to 6, wherein the primary seeds obtained from the primary seed culture are inoculated into the secondary seed culture medium at an inoculum size of 5% to 10% for the secondary seed culture;

preferably, the aeration rate for the secondary seed culture ranges from 0.1 vvm to 1.3 vvm;
preferably, the secondary seed culture is carried out under stirring conditions, with the stirring speed ranging from 150 rpm to 900 rpm;
preferably, the temperature for the secondary seed culture ranges from 30°C to 33°C, and the duration ranges from 8 h to 12 h;
preferably, the secondary seed culture is processed to an OD value of 12 to 16, obtaining the seed liquid.

8. The method according to any one of claims 1 to 7, wherein the carbon source of the fermentation culture medium includes a combination of the second acetate and the ketone compound;

preferably, the second acetate includes any one or a combination of at least two of sodium acetate, potassium acetate and ammonium acetate, further preferably sodium acetate;
preferably, the mass concentration of second acetate in the fermentation culture medium is in a range from 5 g/L to 15 g/L;
preferably, the mass percentage content of ketone compounds in the fermentation culture medium is in a range from 0.1% to 0.6%;
preferably, the fermentation culture medium includes the following components: 5-15 g/L of second acetate, 1%-3% of glucose, 0.6%-2% of yeast powder, 1.5%-3.2% of casein peptone, 0.8%-1.2% of magnesium sulfate, 0.6%-1.4% of thiamine hydrochloride, 0.5%-1% of betaine, 3.5%-6.8% of ammonium sulfate, 5%-7% of potassium dihydrogen phosphate, 2%-4% of disodium hydrogen phosphate and 0.1%-0.6% of ketone compounds;
preferably, the ketone compounds are C3-C10 ketone compounds;
preferably, the ketone compound includes any one or a combination of at least two of acetone, methyl ethyl ketone and cyclohexanone, further preferably acetone;
preferably, in the fermentation culture medium, further includes a second trace element additive, and the usage amount of the second trace element additive is in a range from 0.8% to 2.6%;
preferably, the second trace element additive includes the following components: 4-8 g/L of copper sulfate, 0.12-0.16 g/L of sodium iodide, 5-6 g/L of manganese sulfate, 0.1-0.2 g/L of sodium molybdate, 0.04-0.08 g/L of boric acid, 0.6-0.8 g/L of cobalt chloride, 18-21 g/L of zinc chloride, 38-41 g/L of ferrous sulfate, 0.3-0.5 g/L of biotin and 3-5.2 g/L of sulfuric acid.

9. The method according to any one of claims 1 to 8, wherein the seed liquid is inoculated into the fermentation culture medium at an inoculum size of 3% to 6% for high-density fermentation;
preferably, the nitrogen source supplemented during the high-density fermentation process includes any one or a combination of at least two of urea, ammonium sulfate, ammonium nitrate and ammonia water, further preferably urea.

10. The method according to any one of claims 1 to 9, wherein the temperature for the high-density fermentation ranges from 27°C to 33°C, preferably 28°C to 32°C;

preferably, the pH value for the high-density fermentation ranges from 6.4 to 7.1, further preferably 6.5 to 7;
preferably, the high-density fermentation is carried out under stirring conditions, with the stirring speed ranging from 130 rpm to 800 rpm;
preferably, the aeration rate for the high-density fermentation ranges from 0.5 vvm to 2.4 vvm;
preferably, the high-density fermentation is processed to an OD value 780 to 820, and the fermentation is terminated to obtain the single-cell protein.

11. The method according to any one of claims 1 to 10, wherein the high-density fermentation includes a first fermentation stage, a second fermentation stage and a third fermentation stage, with the OD values increasing sequentially;

the OD value of the first fermentation stage is less than 500, and the carbon to nitrogen ratio of the supplemented acetic acid and nitrogen source in the first fermentation stage is greater than 18;

the OD value of the second fermentation stage is in a range from 500 to 700, and the carbon to nitrogen ratio of the supplemented acetic acid and nitrogen source in the second fermentation stage ranges from 5 to 18;

the OD value of the third fermentation stage is greater than 700, and the carbon to nitrogen ratio of the supplemented acetic acid and nitrogen source in the third fermentation stage is less than 5.

12. The method according to claim 11, wherein the first fermentation stage includes a stage A, a stage B and a stage C carried out sequentially;

in the stage A, the OD value is less than 100, with a temperature ranging from 31°C to 33°C, a pH value of 6.4 to 6.6, an aeration rate of 0.5 vvm to 0.7 vvm, a rotation speed of 130 rpm to 170 rpm, and a carbon to nitrogen ratio of greater than 18 for the supplemented acetic acid and nitrogen source;

in the stage B, $100 \leq$ OD value < 300, with a temperature ranging from 30°C to 32°C, a pH value of 6.5 to 6.7, an aeration rate of 0.9 vvm to 1.1 vvm, a rotation speed of 180 rpm to 220 rpm, and a carbon to nitrogen ratio of greater than 18 for the supplemented acetic acid and nitrogen source;

in the stage C, $300 \leq$ OD value < 500, with a temperature ranging from 29°C to 31°C, a pH value of 6.6 to 6.8, an aeration rate of 1.1 vvm to 1.3 vvm, a rotation speed of 280 rpm to 320 rpm, and a carbon to nitrogen ratio of greater than 18 for the supplemented acetic acid and nitrogen source;

the OD value of the second fermentation stage is in a range from 500 to 700, with a temperature ranging from 28°C to 30°C, a pH value of 6.7 to 6.9, an aeration rate of 1.5 vvm to 1.7 vvm, a rotation speed of 450 rpm to 550 rpm, and a carbon to nitrogen ratio of 5 to 18 for the supplemented acetic acid and nitrogen source;

the OD value of the third fermentation stage is greater than 700, with a temperature ranging from 27°C to 29°C, a pH value of 6.9 to 7.1, an aeration rate of 1.9 vvm to 2.1 vvm, a rotation speed of 650 rpm to 750 rpm, and a carbon to nitrogen ratio of less than 5 for the supplemented acetic acid and nitrogen source.

13. The method according to any one of claims 1 to 12, wherein after the completion of the high-density fermentation, the method further includes a post-treatment step;

preferably, a method of the post-treatment includes subjecting a fermentation broth obtained from the high-density fermentation to solid-liquid separation, washing, and drying in sequence to obtain the single-cell protein;

preferably, a method of the solid-liquid separation is centrifugal separation.

14. The method according to any one of claims 1 to 13, wherein the method specifically includes the following steps:

(1) inoculating the Yarrowia lipolytica strain into the primary seed culture medium and culturing for 16 h to 18 h at a temperature of 30°C to 33°C and a rotation speed of 100 rpm to 300 rpm until the OD value reaches 4.2 to 6.8, obtaining the primary seed;

the primary seed culture medium includes the following components: 5-15 g/L of sodium acetate, 1%-3% of glucose, 0.6%-2% of yeast powder and 1.5%-3.2% of casein peptone;

(2) inoculating the primary seed into the secondary seed culture medium at an inoculum size of 5% to 10%, and culturing for 8 h to 12 h at a temperature of 30°C to 33°C, a rotation speed of 150 rpm to 900 rpm and an aeration rate of 0.1 vvm to 1.3 vvm until the OD value reaches 12 to 16, obtaining the secondary seed, that is, the seed liquid;

the secondary seed culture medium includes the following components: 5-20 g/L of sodium acetate, 1%-3% of glucose, 0.6%-2% of yeast powder, 1.5%-3.2% of casein peptone, 0.8%-2.4% of magnesium sulfate, 0.2%-0.8% of thiamine hydrochloride and 0.8%-2.6% of first trace element additive;

the first trace element additive includes the following components: 4-8 g/L of copper sulfate, 0.12-0.16 g/L of sodium iodide, 5-6 g/L of manganese sulfate, 0.1-0.2 g/L of sodium molybdate, 0.04-0.08 g/L of boric acid, 0.6-0.8 g/L of cobalt chloride, 18-21 g/L of zinc chloride, 38-41 g/L of ferrous sulfate, 0.3-0.5 g/L of biotin and 3-5.2 g/L of sulfuric acid;

(3) inoculating the seed liquid into the fermentation culture medium at an inoculum size of 3% to 6% for high-density fermentation, and supplementing same with acetic acid and urea during the process of high-density fermentation until the OD value reaches 780 to 820, obtaining the single-cell protein;

the fermentation culture medium includes the following components: 5-15 g/L of sodium acetate, 1%-3% of glucose, 0.6%-2% of yeast powder, 1.5%-3.2% of casein peptone, 0.8%-1.2% of magnesium sulfate, 0.6%-1.4% of thiamine hydrochloride, 0.5%-1% of betaine, 3.5%-6.8% of ammonium sulfate, 5%-7% of potassium dihydrogen phosphate, 2%-4% of disodium hydrogen phosphate, 0.1%-0.6% of acetone and 0.8%-2.6% of second trace element additive;

the second trace element additive includes the following components: 4-8 g/L of copper sulfate, 0.12-0.16 g/L of sodium iodide, 5-6 g/L of manganese sulfate, 0.1-0.2 g/L of sodium molybdate, 0.04-0.08 g/L of boric acid, 0.6-0.8 g/L of cobalt chloride, 18-21 g/L of zinc chloride, 38-41 g/L of ferrous sulfate, 0.3-0.5 g/L of biotin, and 3-5.2 g/L of sulfuric acid;

the high-density fermentation includes a first fermentation stage, a second fermentation stage, and a third fermentation stage, with the OD value increasing sequentially;

the OD value of the first fermentation stage is less than 500, with a temperature ranging from 29°C to 33°C, a pH value of 6.5 to 6.8, an aeration rate of 0.5 vvm to 1.3 vvm, a rotation speed of 280 rpm to 320 rpm, and a carbon to nitrogen ratio of greater than 18 for the supplemented acetic acid and urea;

the OD value of the second fermentation stage is in a range from 500 to 700, with a temperature ranging from 28°C to 30°C, a pH value of 6.7 to 6.9, an aeration rate of 1.5 vvm to 1.7 vvm, a rotation speed of 450 rpm to 550 rpm, and a carbon to nitrogen ratio of 5 to 18 for the supplemented acetic acid and nitrogen source;

the OD value of the third fermentation stage is greater than 700, with a temperature ranging from 27°C to 29°C, a pH value of 6.9 to 7.1, an aeration rate of 1.9 vvm to 2.1 vvm, a rotation speed of 650 rpm to 750 rpm, and a carbon to nitrogen ratio of less than 5 for the supplemented acetic acid and nitrogen source.

15. The method according to any one of claims 1 to 14, wherein the yield of the single-cell protein is greater than 2.5 g/(L·h), preferably 6-9 g/(L·h);

preferably, the mass percentage content of crude protein in the single-cell protein is greater than or equal to 35%, further preferably greater than or equal to 55%.

FIG.1

FIG.2

## INTERNATIONAL SEARCH REPORT

| International application No. |
| --- |
| **PCT/CN2023/121022** |

### A. CLASSIFICATION OF SUBJECT MATTER

C12N 1/16(2006.01)i; C12P 7/64(2022.01)i; C12R 1/645(2006.01)n

According to International Patent Classification (IPC) or to both national classification and IPC

### B. FIELDS SEARCHED

Minimum documentation searched (classification system followed by classification symbols)

IPC: C12N, C12P, C12R

Documentation searched other than minimum documentation to the extent that such documents are included in the fields searched

Electronic data base consulted during the international search (name of data base and, where practicable, search terms used)

CNTXT, ENTXT, VEN, CJFD, CNKI, PUBMED, ISI-WEB OF SCIENCE: 高密度发酵, 高密度, 发酵, 单细胞蛋白, 微生物蛋白, 微生物菌体蛋白, 细菌蛋白, 真菌蛋白, 石油蛋白, 甲醇蛋白, 甲烷蛋白, 解脂耶氏酵母, 亚罗解脂酵母, 解脂亚罗酵母, 乙酸盐, 乙酸钠, 乙酸钾, 乙酸铵, 丙酮, 二甲基酮, 甲基乙基酮, 甲乙酮, 丁酮, 环己酮, 培养基, 培养, high density fermentation, high density, fermentation, single cell protein, microbial protein, bacterial protein, fungal protein, mycoprotein, petroleum protein, methanol SCP, yarrowia lipolytica, acetate, sodium acetate, potassium acetate, ammonium acetate, acetone, propanone, butanone, cyclohexanone, medium, media, culture

### C. DOCUMENTS CONSIDERED TO BE RELEVANT

| Category* | Citation of document, with indication, where appropriate, of the relevant passages | Relevant to claim No. |
| --- | --- | --- |
| X | CN 108949591 A (SHANGHAI JITAILAI BIOTECHNOLOGY CO., LTD.) 07 December 2018 (2018-12-07)<br>see claims 1-10, and description, embodiment 1 | 1-15 |
| A | CN 107557309 A (SHANGHAI JITAILAI BIOTECHNOLOGY CO., LTD.) 09 January 2018 (2018-01-09)<br>see abstract | 1-15 |
| A | CN 112553094 A (SHENZHEN KINGSINO TECHNOLOGY CO., LTD.) 26 March 2021 (2021-03-26)<br>see abstract | 1-15 |
| A | CN 114958631 A (WANHUA CHEMICAL (SICHUAN) CO., LTD.; WANHUA CHEMICAL GROUP CO., LTD.) 30 August 2022 (2022-08-30)<br>see abstract | 1-15 |

☑ Further documents are listed in the continuation of Box C.    ☑ See patent family annex.

| | |
| --- | --- |
| \* Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **07 November 2023** | **12 January 2024** |

| Name and mailing address of the ISA/CN | Authorized officer |
| --- | --- |
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
| --- |
| **PCT/CN2023/121022** |

**C.    DOCUMENTS CONSIDERED TO BE RELEVANT**

| Category* | Citation of document, with indication, where appropriate, of the relevant passages | Relevant to claim No. |
| --- | --- | --- |
| A | CN 116731884 A (WANHUA CHEMICAL GROUP CO., LTD.) 12 September 2023 (2023-09-12)<br>see abstract | 1-15 |
| A | CN 1948470 A (BEIJING UNIVERSITY OF CHEMICAL TECHNOLOGY) 18 April 2007 (2007-04-18)<br>see abstract | 1-15 |
| A | WO 9728273 A1 (NORTH AMERICAN VACCINE INC.) 07 August 1997 (1997-08-07)<br>see abstract | 1-15 |
| A | 王晖 等 (WANG, Hui et al.). "解脂耶氏酵母在食品工业中的应用 (Application of Yarrowia lipolytica in food industry)"<br>食品与发酵工业 *(Food and Fermentation Industries)*,<br>Vol. 44, No. 8, 21 March 2018 (2018-03-21), 291-297<br>see page 291, abstract | 1-15 |

Form PCT/ISA/210 (second sheet) (July 2022)

**INTERNATIONAL SEARCH REPORT**
Information on patent family members

International application No.

**PCT/CN2023/121022**

| Patent document cited in search report | | | Publication date (day/month/year) | Patent family member(s) | | | Publication date (day/month/year) |
|---|---|---|---|---|---|---|---|
| CN | 108949591 | A | 07 December 2018 | None | | | |
| CN | 107557309 | A | 09 January 2018 | None | | | |
| CN | 112553094 | A | 26 March 2021 | None | | | |
| CN | 114958631 | A | 30 August 2022 | None | | | |
| CN | 116731884 | A | 12 September 2023 | None | | | |
| CN | 1948470 | A | 18 April 2007 | DK | 1775344 | T3 | 21 November 2011 |
| | | | | AT | 519841 | T | 15 August 2011 |
| | | | | EP | 1775344 | A2 | 18 April 2007 |
| | | | | EP | 1775344 | A3 | 02 May 2007 |
| | | | | EP | 1775344 | B1 | 10 August 2011 |
| WO | 9728273 | A1 | 07 August 1997 | JP | 2001508758 | A | 03 July 2001 |
| | | | | NO | 983474 | D0 | 28 July 1998 |
| | | | | NO | 983474 | L | 30 September 1998 |
| | | | | IL | 125420 | A0 | 12 March 1999 |
| | | | | KR | 19990082265 | A | 25 November 1999 |
| | | | | AU | 2115897 | A | 22 August 1997 |
| | | | | HU | 9901039 | A2 | 28 July 1999 |
| | | | | EP | 0877816 | A1 | 18 November 1998 |
| | | | | PL | 328096 | A1 | 04 January 1999 |

Form PCT/ISA/210 (patent family annex) (July 2022)